# EUROPÄISCHE PATENTSCHRIFT

(11) **EP 0 740 706 B1**
(45) Veröffentlichungstag und Bekanntmachung des Hinweises auf die Patenterteilung: **19.10.2005**
(21) Anmeldenummer: 95906961.8
(22) Anmeldetag: 18.01.1995
(51) Int. Cl.: C12N 15/61, C12N 15/56, C12N 9/90, C12N 9/24, C12N 9/26, C12N 1/21, C12P 19/24

(54) **HERSTELLUNG VON AKARIOGENEN ZUCKERERSATZSTOFFEN**
PRODUCTION OF ACARIOGENIC SUGAR SUBSTITUTES
PRODUCTION DE SUBSTITUTS ACARIOGENES DU SUCRE

(30) Priorität: 19.01.1994 DE 4401451; 22.04.1994 DE 4414185
(43) Veröffentlichungstag der Anmeldung: 06.11.1996
(73) Patentinhaber: Südzucker Aktiengesellschaft Mannheim/Ochsenfurt, 68165 Mannheim (DE)
(72) Erfinder: MATTES, Ralf, D-70619 Stuttgart (DE); KLEIN, Kathrin, D-70197 Stuttgart (DE); SCHIWECK, Hubert, D-67549 Worms (DE); MUNIR, Mohammad, D-67271 Kindenheim (DE); KUNZ, Markwart, D-67550 Worms (DE)
(74) Vertreter: Weiss, Wolfgang
(86) Internationale Anmeldenummer: PCT/EP1995/000165
(87) Internationale Veröffentlichungsnummer: WO 1995/020047

(56) Entgegenhaltungen:
- EP-A- 0 028 900
- EP-A- 0 109 009
- EP-A- 0 200 069
- EP-A- 0 483 755
- ERNST WINNACKER 'From genes to clones:Introduction to gene technology.' , VCH , 1987 siehe Seite 387 - Seite 394

## Beschreibung

Die vorliegende Erfindung betrifft ein verbessertes Verfahren zur Herstellung von nicht-kariogenen Zuckern, insbesondere Trehalulose oder/und Palatinose, unter Verwendung rekombinanter DNA-Technologie.

Die akariogenen Zuckerersatzstoffe Palatinose (Isomaltulose) und Trehalulose werden großtechnisch aus Saccharose durch eine enzymatische Umlagerung unter Verwendung von immobilisierten Bakterienzellen (z.B. der Spezies Protaminobacter rubrum, Erwinia rhapontici, Serratia plymuthica) hergestellt. Dabei wird die zwischen den beiden Monosaccharideinheiten des Disaccharids Saccharose bestehende α1 → β2 glykosidische Bindung zu einer α1 → 6 Bindung bei Palatinose bzw. einer α1 *→* α1 Bindung bei Trehalulose isomerisiert. Diese Umlagerung von Saccharose zu den beiden akariogenen Disacchariden erfolgt unter Katalyse des bakteriellen Enzyms Saccharose-Isomerase, auch Saccharose-Mutase genannt. Je nach verwendetem Organismus ergibt sich bei dieser Reaktion ein Produktgemisch, das neben den erwünschten akariogenen Disacchariden Palatinose und Trehalulose auch gewisse Anteile an unerwünschten Monosacchariden (Glucose oder/und Fructose) enthält. Diese Monosaccharidanteile sind ein erhebliches technisches Problem, da zu ihrer Abtrennung aufwendige Reinigungsprozeduren (meist fraktionierte Kristallisationen) erforderlich sind.

So offenbart EP-A-0 109 009 ein Verfahren zur Herstellung einer Produktlösung, die als Hauptkomponente 1-0-α-D-Glucopyranosido-D-Fructose (Trehalulose) enthält, aus einer Saccharose und Isomaltulose (Palatinose) enthaltenden Lösung mit Hilfe von Palatinose aus Saccharose bildenden Mikroorganismen bzw. Enzymen daraus. Beispiel 3 (S. 9 und 10) offenbart die Verwendung von immobilisierter Saccharose-Isomerase in reiner Form, wobei eine Produktzusammensetzung erhalten wird, die mit der bei Verwendung von vollständigen immobilisierten Zellen gemäß Beispiel 2 erhaltenen Produktzusammensetzung nahezu identisch ist (S. 10, Z. 5-7), insbsondere hinsichtlich des Anteils unerwünschter Nebenprodukte.

EP-A-0 200 069 offenbart ein Verfahren zur Herstellung von Palatinose in einem Bioreaktor unter Verwendung von immobilisierter Saccharose-Isomerase, die aus einem Rohextrakt durch selektive Bindung an eine anionische Trägermatrix gereinigt und immobilisiert worden ist. In Beispiel 1 wird eine Produktzusammensetzung beschrieben, die neben den erwünschten akariogenen Disacchariden Isomaltulose (Palatinose) und 1,1'-Disaccharid (Trehalulose) 2,1 bis 2,5 % Fructose und 0,6 bis 1,0 % Glucose enthält.

EP-A-0 483 755 offenbart ein Verfahren zur Herstellung von Tehalulose und Isomaltulose, wobei eine Saccharose-Lösung mit mindestens einem Trehalulose-bildenden Enzymsystem eines Trehalulose-bildenden Mikroorganismus bei einer Temperatur von 10-35 °C in Kontakt gebracht wird, wobei ein überwiegend Trehalulose enthaltendes Produktgemisch erhalten wird. Bei Inkubationstemperaturen von 15-30 °C werden nur relativ geringe Mengen an Fructose und Glucose (0,2 bis 0,6 %) erhalten. Bei höheren, in großtechnischen Verfahren bevorzugten Inkubationstemperaturen nimmt jedoch der Anteil an Monosacchariden deutlich zu (S. 7, Z. 39-42). Ab 45 °C wird sogar eine rasche thermische Inaktivierung der Enzympräparation gefunden.

Eine der vorliegenden Erfindung zugrundeliegende Aufgabe bestand somit darin, die Bildung von Monosacchariden bei der Isomerisierung von Saccharose zu Trehalulose oder/und Palatinose möglichst weitgehend zu unterdrücken. Eine andere, der vorliegenden Erfindung zugrundeliegende Aufgabe bestand in der Bereitstellung von Organismen, die Palatinose oder/und Trehalulose in höherer Ausbeute als bekannte Organismen erzeugen.

Zur Lösung dieser Aufgaben werden rekombinante DNA-Moleküle, mit rekombinanten DNA-Molekülen transformierte Organismen, rekombinante Proteine sowie ein verbessertes Verfahren zur Herstellung von nicht-kariogenen Zuckern, insbesondere von Palatinose oder/und Trehalulose, bereitgestellt.

Ein Gegenstand der Erfindung ist eine DNA-Sequenz, welche dadurch gekennzeichnet ist, daß sie für ein Protein mit einer Saccharose-Isomerase-Aktivität codiert und
(a) eine der in SEQ ID NO. 1, SEQ ID NO. 2, SEQ ID NO. 3, SEQ ID NO. 9, SEQ ID NO. 11 oder SEQ ID NO. 13 gezeigten Nukleotidsequenzen gegebenenfalls ohne den Signalpeptid-codierenden Bereich,
(b) eine der Sequenzen aus (a) im Rahmen der Degeneration des genetischen Codes entsprechende Nukleotidsequenz, oder
(c) eine mit den Sequenzen aus (a) oder/und (b) hybridisierende Nukleotidsequenz umfaßt.

Im Zusammenhang mit der vorliegenden Erfindung soll der Begriff "Protein mit einer Saccharose-Isomerase-Aktivität" solche Proteine umfassen, die zur Isomerisierung von Saccharose zu anderen Disacchariden in der Lage sind, wobei die α1 → β2 glykosidische Bindung zwischen Glucose und Fructose in der Saccharose in eine andere glykosidische Bindung zwischen zwei Monosaccharideinheiten überführt wird, insbesondere in eine α1 → 6 Bindung oder/und eine α1 → α1 Bindung. Besonders bevorzugt betrifft der Begriff "Protein mit einer Saccharose-Isomerase-Aktivität" daher ein Protein, das zur Isomerisierung von Saccharose zu Palatinose oder/und Trehalulose in der Lage ist. Dabei beträgt der Anteil von Palatinose und Trehalulose an den gesamten Disacchariden, die durch Isomerisierung von Saccharose gebildet werden, vorzugsweise ≥ 2 %, besonders bevorzugt ≥ 20 % und am meisten bevorzugt ≥ 50 %.

Die in SEQ ID NO. 1 gezeigte Nukleotidsequenz codiert für die vollständige Saccharose-Isomerase aus dem Mikroorganismus Protaminobacter rubrum (CBS 547,77) einschließlich des Signalpeptidbereichs. Die in SEQ ID NO. 2 gezeigte Nukleotidsequenz codiert für den N-terminalen Abschnitt der Saccharose-Isomerase aus dem Mikroorganismus Erwinia rhapontici (NCPPB 1578) einschließlich des Signalpeptidbereichs. Die in SEQ ID NO. 3 gezeigte Nukleotidsequenz kodiert für einen Abschnitt der Saccharose-Isomerase aus dem Mikroorganismus SZ 62 (Enterobacter spec.).

Der für das Signalpeptid codierende Bereich der SEQ ID NO. 1 reicht von Nukleotid 1 - 99. In SEQ ID NO. 2 reicht der Signalpeptid-codierende Bereich von Nukleotid 1 - 108. Die DNA-Sequenz gemäß vorliegender Erfindung umfaßt auch die in SEQ ID NO. 1 und SEQ ID NO. 2 gezeigten Nukleotidsequenzen ohne den Signalpeptid-codierenden Bereich, da das Signalpeptid in der Regel nur für die korrekte Lokalisierung des reifen Proteins in einem bestimmten Zellkompartement (z.B. im periplasmatischen Raum zwischen der äußeren und der inneren Membran, in der äußeren Membran oder in der inneren Membran) oder für den extrazellulären Export, nicht aber für die enzymatische Aktivität als solche notwendig ist. Die vorliegende Erfindung umfaßt somit weiterhin auch für das reife Protein (ohne Signalpeptid) codierende Sequenzen in operativer Verknüpfung mit heterologen Signalsequenzen, insbesondere mit prokaryontischen Signalsequenzen, wie etwa bei E.L. Winnacker, Gene und Klone, Eine Einführung in die Gentechnologie, VCH-Verlagsgesellschaft Weinheim, BRD, (1985), S. 256 beschrieben.

Die Nukleotidsequenz SEQ ID NO. 9 codiert für eine Variante der Isomerase aus Protaminobacter rubrum. Die Nucleotidsequenz SEQ ID NO. 11 codiert für die vollständige Isomerase aus dem Isolat SZ 62. Die Nucleotidsequenz SEQ ID NO. 13 codiert für den Großteil der Isomerase aus dem Mikroorganismus MX-45 (FERM 11808 bzw. FERM BP 3619).

Neben den in SEQ ID NO. 1, SEQ ID NO. 2, SEQ ID NO. 3, SEQ ID NO. 9, SEQ ID NO. 11 oder SEQ ID NO. 13 gezeigten Nukleotidsequenzen und einer dieser Sequenzen im Rahmen der Degeneration des genetischen Codes entsprechenden Nukleotidsequenzen umfaßt die vorliegende Erfindung auch noch eine DNA-Sequenz, die mit einer dieser Sequenzen hybridisiert, vorausgesetzt, daß sie für ein Protein codiert, das zur Isomerisierung von Saccharose in der Lage ist. Der Begriff "Hybridisierung" gemäß vorliegender Erfindung wird wie bei Sambrook et al. (Molecular Cloning. A Laboratory Manual, Cold Spring Harbor Laboratory Press (1989), 1.101-1.104) verwendet. Gemäß vorliegender Erfindung spricht man von einer Hybridisierung, wenn nach Waschen für 1 Stunde mit 1 x SSC und 0,1 % SDS bei 55°C, vorzugsweise bei 62°C und besonders bevorzugt bei 68°C, insbesondere für 1 Stunde in 0,2 x SSC und 0,1 % SDS bei 55°C, vorzugsweise bei 62°C und besonders bevorzugt bei 68°C noch ein positives Hybridisierungssignal beobachtet wird. Eine unter derartigen Waschbedingungen mit einer der in SEQ ID NO:1 oder SEQ ID NO:2 gezeigten Nukleotidsequenzen oder einer damit im Rahmen der Degeneration des genetischen Codes entsprechenden Nukleotidsequenz hybridisierende Nukleotidsequenz ist eine erfindungsgemäße Nukleotidsequenz.

Vorzugsweise weist die erfindungsgemäße DNA-Sequenz
(a) eine der in SEQ ID NO. 1, SEQ ID NO. 2, SEQ ID NO. 3, SEQ ID NO. 9, SEQ ID NO. 11 oder SEQ ID NO. 13 gezeigten Nukleotidsequenzen gegebenenfalls ohne den Signalpeptid-codierenden Bereich oder
(b) eine zu den Sequenzen aus (a) mindestens 70 % homologe Nukleotidsequenz auf.

Vorzugsweise weist die erfindungsgemäße DNA-Sequenz auch eine mindestens 80 % homologe Nukleotidsequenz zu den konservierten Teilbereichen der in SEQ ID NO. 1, SEQ ID NO. 2, SEQ ID NO. 3, SEQ ID NO. 9, SEQ ID NO. 11 oder SEQ ID NO. 13 gezeigten Nukleotidsequenzen auf. Diese konservierten Teilbereiche sind insbesondere von Nukleotid 139 - 186, Nukleotid 256 - 312, Nukleotid 328 - 360, Nukleotid 379 - 420 oder/und Nukleotid 424 - 444 der in SEQ ID NO. 1 gezeigten Nukleotidsequenz.

In einer besonders bevorzugten Ausführungsform weist die erfindungsgemäße DNA-Sequenz eine mindestens 80 % homologe, insbesondere eine mindestens 90 % homologe Nukleotidsequenz zu den Teilbereichen
(a) Nukleotid 139 - 155 oder/und
(b) Nukleotid 625 - 644
der in SEQ ID NO. 1 gezeigten Nukleotidsequenz auf.

Oligonukleotide, die aus den obigen Sequenzbereichen abgeleitet sind, haben sich als Primer zur PCR-Amplifikation von Isomerase-Fragmenten aus der genomischen DNA einer Vielzahl getesteter Mikroorganismen, z.B. Protaminobacter rubrum (CBS 547, 77), Erwinia rhapontici (NCPPB 1578), Isolat SZ 62 und Pseudomonas mesoacidophila MX-45 (FERM 11808) bewährt.

Besonders bevorzugt werden zu diesem Zweck die folgenden Oligonukleotide, gegebenenfalls in Form von Gemischen verwendet, wobei die in Klammern befindlichen Basen alternativ vorhanden sein können:

Oligonukleotid I (17 nt):
5'-TGGTGGAA(A,G)GA(G,A)GCTGT-3'

Oligonukleotid II (20 nt):
5'-TCCCAGTTCAG(G,A)TCCGGCTG-3'

Das Oligonukleotid I ist aus den Nukleotiden 139-155 von SEQ ID NO. 1 abgeleitet und das Oligonukleotid II ist aus der zu den Nukleotiden 625 - 644 komplementären Sequenz von SEQ ID NO. 1 abgeleitet. Die Unterschiede zwischen den homologen Teilbereichen der erfindungsgemäßen DNA-Sequenzen und den als Oligonukleotid I und Oligonukleotid II bezeichneten Sequenzen sind vorzugsweise jeweils maximal 2 Nukleotide und besonders bevorzugt jeweils maximal 1 Nukleotid.

In einer weiteren besonders bevorzugten Ausführungsform der vorliegenden Erfindung weist die DNA-Sequenz eine mindestens 80 % homologe, insbesondere eine mindestens 90 % homologe Nukleotidsequenz zu den Teilbereichen von
(c) Nukleotid 995 - 1013 oder/und
(d) Nukleotid 1078 - 1094
der in SEQ ID NO. 1 gezeigten Nukleotidsequenz auf.

Oligonukleotide, die aus den obigen Sequenzbereichen abgeleitet sind, hybridisieren mit Saccharose-Isomerasegenen aus den Organismen Protaminobacter rubrum und Erwinia rhapontici. Besonders bevorzugt werden die folgenden Oligonukleotide, gegebenenfalls in Form von Gemischen, verwendet, wobei die in Klammern angegebenen Basen alternativ vorhanden sein können:

Oligonukleotid III (19 nt):
AAAGATGGCG(G,T)CGAAAAGA

Oligonukleotid IV (17 nt):
5'-TGGAATGCCTT(T,C)TTCTT-3'

Oligonukleotid III ist aus den Nukleotiden 995 - 1013 von SEQ ID NO. 1 abgeleitet und Oligonukleotid IV ist aus den Nukleotiden 1078 - 1094 von SEQ ID NO. 1 abgeleitet. Die Unterschiede zwischen den homologen Teilbereichen der erfindungsgemäßen DNA-Sequenzen und den als Oligonukleotid III und IV bezeichneten Sequenzen sind vorzugsweise jeweils maximal 2 Nukleotide und besonders bevorzugt jeweils maximal 1 Nukleotid.

Erfindungsgemäße Nukleotidsequenzen sind insbesondere aus Mikroorganismen der Gattungen Protaminobacter, Erwinia, Serratia, Leuconostoc, Pseudomonas, Agrobacterium und Klebsiella erhältlich. Spezifische Beispiele für solche Mikroorganismen sind Protaminobacter rubrum (CBS 547,77), Erwinia rhapontici (NCPPB 1578), Serratia plymuthica (ATCC 15928), Serratia marcescens (NCIB 8285), Leuconostoc mesenteroides NRRL B-521f (ATCC 10830a), Pseudomonas mesoacidophila MX-45 (FERM 11808 bzw. FERM BP 3619) Agrobacterium radiobacter MX-232 (FERM 12397 bzw. FERM BP 3620), Klebsiella subspezies und Enterobacter spezies. Die erfindungsgemäßen Nukleotidsequenzen können auf einfache Weise aus dem Genom der betreffenden Mikrooganismen z.B. unter Verwendung von Oligonukleotiden aus einem oder mehreren der konservierten Bereiche von SEQ ID NO. 1, SEQ ID NO. 2, SEQ ID NO. 3, SEQ ID NO. 9, SEQ ID NO. 11 und SEQ ID NO. 13 durch Amplifikations- oder/und Hybridisierungs-Standardtechniken isoliert und charakterisiert werden. Vorzugsweise werden die erfindungsgemäßen Nukleotidsequenzen durch eine PCR-Amplifikation der genomischen DNA des betreffenden Organismus unter Verwendung der Oligonucleotide I und II gewonnen. Auf diese Weise erhält man ein Teilfragment des betreffenden Saccharose-Isomerasegens, das anschließend als Hybridisierungssonde zur Isolierung des kompletten Gens aus einer Genbank des betreffenden Mikroorganismus verwendet werden kann. Alternativ können die Nukleotidsequenzen durch Herstellung einer Genbank aus dem jeweiligen Organismus und direkte Musterung dieser Genbank mit den Oligonukleotiden I, II, III oder/und IV gewonnen werden.

Ein weiterer Gegenstand der vorliegenden Erfindung ist ein Vektor, der mindestens eine Kopie einer erfindungsgemäßen DNA-Sequenz enthält. Dieser Vektor kann ein beliebiger prokaryontischer oder eukaryontischer Vektor sein, auf dem die erfindungsgemäße DNA-Sequenz sich vorzugsweise unter Kontrolle eines Expressionssignals (Promotor, Operator, Enhancer etc.) befindet. Beispiele für prokaryontische Vektoren sind chromosomale Vektoren wie etwa Bacteriophagen (z.B. Bacteriophage λ) und extrachromosomale Vektoren wie etwa Plasmide, wobei zirkuläre Plasmidvektoren besonders bevorzugt sind. Geeignete prokaryontische Vektoren sind z.B. bei Sambrook et al., supra, Kapitel 1 - 4 beschrieben.

Ein besonders bevorzugtes Beispiel eines erfindungsgemäßen Vektors ist das Plasmid pHWS 88, welches ein Saccharose-Isomerase-Gen von Protaminobacter rubrum unter Kontrolle des regulierbaren tac-Promotors trägt. Das Plasmid pHWS 88 wurde am 16. Dezember 1993 bei der Deutschen Sammlung von Mikroorganismen und Zellkulturen (DSM), Mascheroder Weg 1b, 38124 Braunschweig, Bundesrepublik Deutschland unter der Hinterlegungsnummer DSM 8824 gemäß den Vorschriften des Budapester Vertrages hinterlegt.

In einer weiteren bevorzugten Ausführungsform der vorliegenden Erfindung ist der erfindungsgemäße Vektor ein Plasmid, das in der Wirtszelle mit einer Kopienzahl von weniger als 10, besonders bevorzugt mit einer Kopienzahl von 1 bis 2 Kopien pro Wirtszelle vorliegt. Beispiele solcher Vektoren sind einerseits chromosomale Vektoren, wie etwa Bacteriophage λ oder F-Plasmide. Die Herstellung von F-Plasmiden, die das Saccharose-Isomerasegen enthalten, kann beispielsweise durch Transformation eines F-Plasmid-haltigen E.coli-Stamms mit einem, das Saccharose-Isomerasegen enthaltenden Transposon und anschliessende Selektion auf rekombinante Zellen erfolgen, in denen das Transposon in das F-Plasmid integriert hat. Ein Beispiel für ein derartiges rekombinantes Transposon ist das Plasmid pHWS 118, welches das Transposon Tn 1721 Tet enthält und durch Klonierung eines das Saccharose-Isomerase-Gen enthaltenden DNA-Fragments aus dem oben beschriebenen Plasmid pHWS 88 in das Transposon pJOE 105 (DSM 8825) hergestellt wurde.

Andererseits kann der erfindungsgemäße Vektor auch ein eukaryontischer Vektor sein, z.B. ein Hefevektor (z.B. YIp, YEp etc.) oder ein für höhere Zellen geeigneter Vektor (z.B. ein Plasmidvektor, viraler Vektor, Pflanzenvektor) sein. Derartige Vektoren sind dem Fachmann auf dem Gebiet der Molekularbiologie geläufig, so daß hier nicht näher darauf eingegangen werden muß. Insbesondere wird in diesem Zusammenhang auf Sambrook et al., supra, Kapitel 16 verwiesen.

Ein weiterer Gegenstand der vorliegenden Erfindung ist eine Zelle, die mit einer erfindungsgemäßen DNA-Sequenz oder einem erfindungsgemäßen Vektor transformiert ist. In einer Ausführungsform ist diese Zelle eine prokaryontische Zelle, vorzugsweise eine gram-negative prokaryontische Zelle, besonders bevorzugt eine Enterobakterienzelle. Dabei kann einerseits eine Zelle verwendet werden, die kein eigenes Saccharose-Isomerasegen enthält, wie z.B. E.coli, andererseits können aber auch Zellen verwendet werden, die bereits ein solches Gen auf ihrem Chromosom enthalten, z.B. die oben als Quelle für Saccharose-Isomerasegene genannten Mikroorganismen. Bevorzugte Beispiele für geeignete prokaryontische Zellen sind E.coli-, Protaminobacter rubrum- oder Erwinia rhapontici-Zellen. Die Transformation prokaryontischer Zellen mit exogenen Nukleinsäuresequenzen ist einem Fachmann auf dem Gebiet der Molekularbiologie geläufig (vgl. z.B. Sambrook et al., supra, Kapitel 1 - 4).

In einer weiteren Ausführungsform der vorliegenden Erfindung kann die erfindungsgemäße Zelle jedoch auch eine eukaryontische Zelle sein, wie etwa eine Pilzzelle (z.B. Hefe), eine tierische oder eine pflanzliche Zelle. Verfahren zur Transformation bzw. Transfektion eukaryontischer Zellen mit exogenen Nukleinsäuresequenzen sind dem Fachmann auf dem Gebiet der Molekularbiologie ebenfalls geläufig und brauchen hier nicht näher erläutert zu werden (vgl. z.B. Sambrook et al., Kapitel 16).

Die Erfindung betrifft auch ein Protein mit einer Saccharose-Isomerase-Aktivität wie oben definiert, das von einer erfindungsgemäßen DNA-Sequenz codiert ist. Vorzugsweise umfaßt dieses Protein
(a) eine der in SEQ ID NO. 4, SEQ ID NO. 5, SEQ ID NO. 6, SEQ ID NO. 10, SEQ ID NO. 12 oder SEQ ID NO. 14 gezeigten Aminosäuresequenzen gegebenenfalls ohne den Signalpeptid-bereich oder
(b) eine zu den Sequenzen aus (a) mindestens 80 % homologe Aminosäuresequenz.

Die in SEQ ID NO. 4 gezeigte Aminosäuresequenz umfaßt die vollständige Saccharose-Isomerase aus Protaminobacter rubrum. Das Signalpeptid reicht von Aminosäure 1 - 33. Das reife Protein beginnt mit Aminosäure 34. Die in SEQ ID NO. 5 gezeigte Aminosäuresequenz umfaßt den N-terminalen Abschnitt der Saccharose-Isomerase aus Erwinia rhapontici. Das Signalpeptid reicht von Aminosäure 1 - 36. Das reife Protein beginnt mit Aminosäure 37. Die in SEQ ID NO. 6 gezeigte Aminosäuresequenz umfaßt einen Abschnitt der Saccharose-Isomerase aus dem Mikroorganismus SZ 62. In Fig. 1 ist ein Vergleich der Aminosäuresequenzen der Isomerasen aus P. rubrum, E. rhapontici und SZ 62 gezeigt.

Die Aminosäuresequenz SEQ ID NO. 10 umfaßt eine Variante der Isomerase aus P. rubrum. Die Aminosäuresequenz SEQ ID NO. 12 umfaßt die vollständige Isomerase aus SZ 62. Dieses Enzym hat eine hohe Aktivität bei 37°C und produziert nur einen sehr geringen Anteil an Monosacchariden. Die Aminosäuresequenz SEQ ID NO. 14 umfaßt einen Großteil der Isomerase aus MX-45. Dieses Enzym produziert ca. 85% Trehalulose und 13% Palatinose.

Besonders bevorzugt weist das erfindungsgemäße Protein eine mindestens 90 % homologe Aminosäuresequenz zu konservierten Teilbereichen aus den in SEQ ID NO. 4, SEQ ID NO. 5,SEQ ID NO. 6, SEQ ID NO. 10, SEQ ID NO. 12 oder SEQ ID NO. 14 gezeigten Aminosäuresequenzen auf, insbesondere in Teilbereichen von
(a) Aminosäure 51 - 149,
(b) Aminosäure 168 - 181,
(c) Aminosäure 199 - 250,
(d) Aminosäure 351 - 387 oder/und
(e) Aminosäure 390 - 420
der in SEQ ID NO.4 gezeigten Aminosäuresequenz auf.

Mittels der oben genannten DNA-Sequenzen, Vektoren, transformierten Zellen und Proteinen ist die Bereitstellung einer Saccharose-Isomerase-Aktivität ohne störende enzymatische Nebenaktivitäten auf einfache Weise möglich.

Hierzu kann einerseits die Saccharose-Isomerase durch rekombinante DNA-Technologie als Bestandteil eines Extrakts aus dem Wirtsorganismus oder in isolierter und gereinigter Form (z.B. durch Expression in E.coli) erhalten werden. Dieses vorzugsweise gereinigte und isolierte Saccharose-Isomerase-Enzym kann z.B. in immobilisierter Form zur technischen Herstellung von akariogenen Zuckern wie etwa Trehalulose oder/und Palatinose durch Umsetzung von Saccharose in einem Enzymreaktor verwendet werden. Die Immobilisierung von Enzymen ist einem Fachmann geläufig und muß an dieser Stelle nicht ausführlich beschrieben werden.

Andererseits kann die Herstellung von akariogenen Zuckern aus Saccharose auch in einem vollständigen Mikroorganismus, vorzugsweise in immobilisierter Form, erfolgen. Durch Klonierung des oben genannten Saccharose-Isomerasegens in einen Organismus ohne bzw. mit reduziertem Palatinose- oder/und Trehalulosestoffwechsel (d.h. in einem Organismus, der nicht in der Lage ist, die oben genannten Zucker signifikant abzubauen) kann ein neuer Organismus erzeugt werden, der durch Einführung exogener DNA zur Herstellung von akariogenen Disacchariden ohne nennenswerte Bildung von Monosacchariden in der Lage ist. Zur Einführung des Saccharose-Isomerasegens eignet sich somit einerseits ein Organismus, der Palatinose oder/und Trehalulose nicht verwerten kann (z.B. E.coli, Bacillus, Hefe) und andererseits ein Organismus, der grundsätzlich zur Verwertung von Palatinose oder/und Trehalulose in der Lage wäre, aber durch ungezielte oder gezielte Mutation einen reduzierten Palatinose- oder/und Trehalulosestoffwechsel aufweist.

Der Begriff "reduzierter Palatinose- oder/und Trehalulosestoffwechsel" bedeutet im Sinne der vorliegenden Erfindung, daß eine ganze Zelle des betreffenden Organismus bei der Verwertung von Saccharose als C-Quelle akariogene Disaccharide erzeugt und diese aber nur in geringem Umfang metabolisch verwerten kann, z.B. indem sie zu Monosacchariden abgebaut werden. Vorzugsweise erzeugt der Organismus weniger als 2,5 %, besonders bevorzugt weniger als 2 %, am meisten bevorzugt weniger als 1 % Glucose plus Fructose auf Basis der Summe von akariogenen Disacchariden und Monosaccharidabbauprodukten bei einer Temperatur von 15 - 65°C, insbesondere von 25 - 55°C.

Ein weiterer Gegenstand der vorliegenden Erfindung ist somit eine Zelle, die mindestens eine für ein Protein mit einer Saccharose-Isomerase-Aktivität codierende DNA-Sequenz enthält und einen wie oben definierten reduzierten Palatinose- oder/und Trehalulosestoffwechsel aufweist. Eine derartige Zelle erzeugt höhere Anteile der nicht-kariogenen Disaccharide Trehalulose oder/und Palatinose und verringerte Mengen an den störenden Nebenprodukten Glucose bzw. Fructose.

In einer Ausführungsform der vorliegenden Erfindung kann die Reduzierung des Palatinose- oder/und Trehalulosestoffwechsels durch teilweise oder vollständige Hemmung der Expression von Invertase- oder/und Palatinasegenen erfolgen, die für den intrazellulären Abbau von Palatinose oder/und Trehalulose verantwortlich sind. Diese Hemmung der Genexpression kann beispielsweise durch zielgerichtete Mutagenese oder/und Deletion der betreffenen Gene erfolgen. Eine zielgerichtete Mutation des in SEQ ID NO. 7 gezeigten Palatinasegens oder des in SEQ ID NO. 15 gezeigten Palatinose-Hydrolase-Gens kann beispielsweise durch Einführung eines zur homologen chromosomalen Rekombination geeigneten Vektors, der ein mutiertes Palatinasegen trägt, und Selektion von Organismen erfolgen, in denen eine derartige Rekombination stattgefunden hat. Das Prinzip der Selektion durch genetische Rekombination wird bei E.L. Winnacker, Gene und Klone, Eine Einführung in die Gentechnologie (1985), VCH-Verlagsgesellschaft Weinheim, BRD, S. 320 ff. erläutert.

Weiterhin können erfindungsgemäße Organismen mit reduziertem Palatinose- oder/und Trehalulosestoffwechsel auch durch unspezifische Mutagenese aus geeigneten Ausgangsorganismen und Selektion der Palatinase-Defektmutanten erhalten werden. Ein Beispiel für eine derartige Palatinase-Defektmutante ist der Protaminobacter rubrum-Stamm SZZ 13, der am 29. März 1994 bei der Deutschen Sammlung von Mikroorganismen und Zellkulturen (DSM), Mascheroder Weg 1b, 38124 Braunschweig, Bundesrepublik Deutschland, unter der Hinterlegungsnummer DSM 9121 gemäß den Vorschriften des Budapester Vertrags hinterlegt wurde. Dieser Mikroorganismus wurde durch unspezifische Mutagenese von P. rubrum-Wildtypzellen mit N-Methyl-N'-nitro-N-nitrosoguanidin hergestellt und zeichnet sich dadurch aus, daß er die nicht-kariogenen Zucker Trehalulose und Palatinose nicht mehr in Glucose und Fructose spalten kann. Die Selektion solcher Mutanten kann z.B. durch Verwendung von MacConkey-Palatinase-Medien oder Minimalsalzmedien mit Palatinose oder Glucose als einziger C-Quelle erfolgen. Die Mutanten, die auf MacConkey-Palatinose-Medium (MacConkey-Agar-Base von Difco Laboratories, Detroit, Michigan, USA (40 g/l) und 20 g/l Palatinose) weiß sind oder auf Minimalsalzmedien mit Glucose als einziger C-Quelle, aber nicht auf entsprechenden Medien mit Palatinose als einziger C-Quelle wachsen, werden als Palatinase-DefektMutanten identifiziert.

weiterhin betrifft die vorliegende Erfindung ein Verfahren zur Isolierung von Nukleinsäuresequenzen, die für ein Protein mit einer Saccharose-Isomerase-Aktivität codieren, wobei man eine Genbank aus einem Spenderorganismus, der eine für ein Protein mit einer Saccharose-Isomerase-Aktivität codierende DNA-Sequenz enthält, in einem geeigneten Wirtsorganismus anlegt, die Klone der Genbank untersucht und die Klone isoliert, die eine für ein Protein mit Saccharose-Isomerase-Aktivität codierende Nukleinsäure enthalten. Die auf diese Weise isolierten, für Saccharose-Isomerase codierenden Nukleinsäuren können wiederum zur Einführung in Zellen wie oben beschrieben verwendet werden, um neue Produzentenorganismen von akariogenen Zuckern bereitzustellen.

Bei diesem Verfahren wählt man als Wirtsorganismus vorzugsweise einen Organismus, der keine eigenen funktionellen Gene für den Palatinosestoffwechsel, insbesondere keine funktionellen Palatinase- oder/und Invertasegene besitzt. Ein bevorzugter Wirtsorganismus ist E.coli. Zur Erleichterung der Charakterisierung von Palatinose-produzierenden Klonen können bei der Untersuchung der Klone der Genbank Saccharose-spaltende Klone.und die darin enthaltenen, vom Spenderorganismus stammenden DNA-Sequenzen isoliert und in einem Galactose nicht verwertenden E.coli-Stamm transformiert werden, der als Screeningstamm für die Klone der Genbank eingesetzt wird.

Andererseits kann die Untersuchung der Klone der Genbank auf DNA-Sequenzen, die für ein Protein mit einer Saccharose-Isomerase-Aktivität codieren, auch unter Verwendung von Nukleinsäuresonden erfolgen, die aus den Sequenzen SEQ ID NO. 1, SEQ ID NO. 2, SEQ ID NO. 3, SEQ ID NO. 9, SEQ ID NO. 11 bzw. SEQ ID NO. 13 abgeleitet sind, die für die Saccharose-Isomerase-Gene aus Protaminobacter rubrum, Erwinia rhapontici und dem Isolat SZ 62 codieren. Besonders bevorzugt verwendet man als Sonden ein durch PCR-Reaktion mit den Oligonukleotiden I und II als Primer gewonnenes DNA-Fragment bzw. die Oligonukleotide III oder/und IV.

Ein weiterer Gegenstand der vorliegenden Erfindung ist ein Verfahren zur Herstellung von nicht-kariogenen Zuckern, insbesondere Trehalulose oder/und Palatinose, welches dadurch gekennzeichnet ist, daß man zur Produktion der Zucker
(a) ein Protein mit Saccharose-Isomerase-Aktivität in isolierter Form,
(b) einem Organismus, der mit einer DNA-Sequenz, die für Protein mit Saccharose-Isomerase-Aktivität codiert, oder einem, mindestens eine Kopie dieser DNA-Sequenz enthaltenden Vektor transformiert ist,
(c) einen Organismus, der mindestens eine für ein Protein mit einer Saccharose-Isomerase-Aktivität codierende DNA-Sequenz enthält und einen reduzierten Palatinose- oder/und Trehalulosestoffwechsel aufweist, oder/und
(d) einen Extrakt aus einer derartigen Zelle bzw. einem derartigen Organismus verwendet.

Die Durchführung des Verfahrens erfolgt im allgemeinen dadurch, daß man das Protein, den Organismus bzw. den Extrakt in einem geeigneten Medium mit Saccharose unter solchen Bedingungen in Kontakt bringt, bei denen die Saccharose durch die Saccharose-Isomerase mindestens teilweise in akariogene Disaccharide umgewandelt wird. Anschließend werden die akariogenen Disaccharide aus dem Medium oder dem Organismus gewonnen und auf bekannte Weise aufgereinigt.

In einer bevorzugten Ausführungsform dieses Verfahrens wird der Organismus, das Protein oder der Extrakt in immobilisierter Form verwendet. Die Immobilisierung von Proteinen (in reiner Form oder in Extrakten) erfolgt vorzugsweise über Kopplung von reaktiven Seitengruppen (z.B. NH₂-Gruppen) an einen geeigneten Träger. Die Immobilisierung von Zellen erfolgt z.B. in einer Natriumalginat/Calciumchlorid-Lösung. Ein überblick über geeignete Methoden zur Immobilisierung von Zellen und Proteinen wird z.B. bei I. Chibata (Immobilized Enzymes, John Wiley and Sons, New York, London, 1978) gegeben.

Bei Verwendung einer mit der Saccharose-Isomerase-Gen transformierten Zelle kann die Produktionsrate von akariogenen Zukkern gegenüber bekannten Organismen durch Erhöhung der Anzahl von Genkopien in der Zelle oder/und durch Erhöhung der Expressionsrate bei einer Kombination mit starken Promotoren steigern. Weiterhin kann durch Transformation einer Zelle, die nicht oder nur in beschränktem Ausmaß zur Verwertung von akariogenen Zuckern in der Lage ist, mit dem Saccharose-Isomerase-Gen eine transformierte Zelle erzeugt werden, mit deren Hilfe akariogene Zucker, insbesondere Palatinose oder/und Trehalulose, ohne oder mit weniger Nebenprodukten gewonnen werden können.

Bei Verwendung eines Mikroorganismus mit reduziertem Palatinose- oder/und Trehalulosestoffwechsel, der bereits ein funktionelles Saccharose-Isomerase-Gen enthält, ist die Transformation mit einem exogenen Saccharose-Isomerase-Gen nicht essentiell, kann aber zur Ausbeuteverbesserung durchgeführt werden.

Eine erfindungsgemäße Zelle mit reduziertem Palatinoseoder/und Trehalulose-Stoffwechsel kann - wie zuvor ausgeführt - durch teilweise oder vollständige Hemmung der Expression von Palatinasegenen hergestellt werden.

Eine DNA-Sequenz, die für ein Protein mit Palatinase- bzw. Palatinose-Hydrolase-Aktivität codiert, kann
(a) eine der in SEQ ID NO. 7 oder SEQ ID NO. 15 gezeigten Nukleotidsequenzen,
(b) eine der Sequenz aus (a) im Rahmen der Degeneration des genetischen Codes entsprechende Nukleotidsequenz oder
(c) eine mit den Sequenzen aus (a) oder/und (b) hybridisierende Nukleotidsequenz umfassen.

Die DNA-Sequenzen oder ein Vektor, der mindestens eine Kopie der oben genannten DNA-Sequenzen enthält, kann zur Transformation einer Zelle verwendet werden. Ein Protein mit Palatinase-Aktivität, das von einer DNA-Sequenz, wie oben angegeben, codiert ist, weist vorzugsweise eine der in SEQ ID NO. 8 oder SEQ ID NO. 16 gezeigten Aminosäuresequenzen auf.

Die in SEQ ID NO. 8 gezeigte Palatinase aus P. rubrum unterscheidet sich von bekannten Saccharose-spaltenden Enzymen dadurch, daß sie die von bekannten Enzymen nicht gespaltenen Saccharose-Isomere, insbesondere Palatinose, spaltet.

Die in SEQ ID NO. 16 gezeigte Aminosequenz umfaßt eine Palatinose-Hydrolase aus MX-45, die Palatinose unter Bildung von Fructose und Glucose spaltet. Das für dieses Enzym codierende Gen ist in SEQ ID NO. 15 dargestellt und ist im Genom von MX-45 5'-seitig des in SEQ ID NO. 13 gezeigten Isomerasegens lokalisiert. Weiterhin wird die Erfindung durch folgende Sequenzprotokolle und Figuren beschrieben:
SEQ ID NO.1 zeigt die Nukleotidsequenz des für die Saccharose-Isomerase aus Protaminobacter rubrum codierenden Gens. Die für das Signalpeptid codierende Sequenz endet bei Nukleotid Nr. 99.
SEQ ID NO.2 zeigt den N-terminalen Abschnitt der Nukleotidsequenz des für die Saccharose-Isomerase von Erwinia rhapontici codierenden Gens. Die für das Signalpeptid codierende Sequenz endet bei dem Nukleotid mit der Nr. 108.
SEQ ID NO.3 zeigt einen Abschnitt der Nukleotidsequenz des für die Saccharose-Isomerase aus dem Isolat SZ 62 codierenden Gens.
SEQ ID NO.4 zeigt die Aminosäuresequenz der Saccharose-Isomerase aus Protaminobacter rubrum.
SEQ ID NO.5 zeigt den N-terminalen Abschnitt der Aminosäuresequenz der Saccharose-Isomerase aus Erwinia rhapontici.
SEQ ID NO.6 zeigt einen Abschnitt der Aminosäuresequenz der Saccharose-Isomerase aus dem Isolat SZ 62.
SEQ ID NO.7 zeigt die Nukleotidsequenz für das Palatinasegen aus Protaminobacter rubrum.
SEQ ID NO.8 zeigt die Aminosäuresequenz der Palatinase aus Protaminobacter rubrum.
SEQ ID NO.9 zeigt die Nucleotidsequenz einer Variante des Saccharose-Isomerasegens aus P. rubrum.
SEQ ID NO. 10 zeigt die korrespondierende Aminosäuresequenz.
SEQ ID NO. 11 zeigt die vollständige Nucleotidsequenz des Saccharose-Isomerasegens aus SZ 62.
SEQ ID NO. 12 zeigt die korrespondierende Aminosäuresequenz.
SEQ ID NO. 13 zeigt den Großteil des Saccharose-Isomerasegens aus Pseudomonas mescacidophila (MX-45).
SEQ ID NO. 14 zeigt die korrespondierende Aminosäuresequenz.
SEQ ID NO. 15 zeigt das Palatinose-Hydrolase-Gen aus Pseudomonas mesoacidophila (MX-45).
SEQ ID NO. 16 zeigt die korrespondierende Aminosäuresequenz.
Fig. 1 zeigt einen Vergleich der Aminosäuresequenzen zwischen den Saccharose-Isomerasen aus Protaminobacter rubrum, Erwinia rhapontici und dem Isolat SZ 62,
Fig. 2 das Klonierungsschema zur Herstellung des rekombinanten Plasmids pHWS 118, das das Saccharose-Isomerase-Gen auf dem Transposon Tn 1721 enthält,
Fig. 3 das Schema zur Herstellung von E.coli-Transkonjuganten, die das Saccharose-Isomerase-Gen auf einem F-Plasmid enthalten und
Fig. 4 zeigt einen Vergleich zwischen den von P. rubrum-Wildtypzellen und Zellen der P. rubrum Mutante SZZ 13 erzeugten Sacchariden.

Die folgenden Beispiele dienen zur Veranschaulichung der vorliegenden Erfindung.

### BEISPIEL 1

Isolierung des Saccharose-Isomerase-Gens aus Protaminobacter rubrum

Gesamt-DNA aus dem Organismus Protaminobacter rubrum (CBS 574, 77) wurde partiell mit Sau3A I verdaut. Aus dem resultierenden Fragmentgemisch wurden Fragmentscharen der Größe von ca. 10 kBp durch Elution nach gelelektrophoretischer Auftrennung gewonnen und in ein mit BamHI geöffnetes Derivat des Lambda EMBL4-Vektor-Derivats λ RESII (J. Altenbuchner, Gene 123 (1993), 63-68) ligiert. Durch Transfektion von E.coli und Umwandlung der Phagen in Plasmide gemäß obigem Zitat wurde eine Genbank hergestellt. Ein Screening der Kanamycin-resistenten Kolonien dieser Genbank erfolgte mit dem aus der Sequenz des N-Terminus der reifen Isomerase abgeleiteten radioaktiv markierten Oligonukleotids S214 durch Hybridisierung:

S214: 5'-ATCCCGAAGTGGTGGAAGGAGGC-3'
T A A A A

Anschließend erfolgte eine Isolierung der Plasmid-DNA aus den positiv reagierenden Kolonien nach entsprechender Kultivierung. Aus einem auf diese Weise erhaltenen Plasmid pKAT 01 wurde nach Erstellung einer Restriktionskarte geeignete Subfragmente sequenziert und somit die komplette, in SEQ ID NO. 1 gezeigte Nukleotidsequenz der für die Isomerase codierenden DNA erhalten. Die davon abgeleitete Aminosäuresequenz entspricht vollständig der durch Sequenzierung (Edmann-Abbau) gewonnenen Peptidsequenz der reifen Isomerase. Im nicht-codierenden 3'-Bereich dieses Isomerasegens befindet sich eine Schnittstelle für SacI, im nicht-codierenden 5'-Bereich eine Schnittstelle für HindIII. Dies ermöglicht die Subklonierung des intakten Isomerasegens in den Vektor pUCBM 21 (Derivat des Vektors pUC 12, Boehringer Mannheim GmbH, Mannheim, Deutschland), der zuvor mit den genannten Enzymen vorgespalten worden war. Das resultierende Plasmid erhielt die Bezeichnung pHWS 34.2 und verleiht den es tragenden E.coli-Zellen die Fähigkeit zur Synthese von Saccharose-Isomerase.

Eine Variante des Saccharose-Isomerasegens aus P. rubrum besitzt die in SEQ ID N0. 9 gezeigte Nucleotidsequenz.

### BEISPIEL 2

Klonierung und Expression der Saccharose-Isomerase aus P. rubrum in E.coli

### 1. Herstellung des Plasmids pHWS88

Aus dem Plasmid pHWS 34.2 wurde durch Verwendung eines Oligonukleotids S434 mit der Sequenz 5'-CGGAATTCTTATGCCCCGTCAAGGA-3' der nicht-codierende 5'-Bereich des Saccharose-Isomerase-gens unter gleichzeitiger Einfügung einer EcoRI-Schnittstelle (GAATTC) entfernt. Das so entstehende Derivat des Isomerase-gens wurde mit BstE II behandelt, das überhängende BstE II-Ende mit S1-Nuklease abgedaut und anschließend mit EcoRI nachverdaut. Das auf diese Weise behandelte Isomerasegen wurde in den mit EcoRI und SmaI vorbehandelten Vektor pBTacI (Boehringer Mannheim GmbH, Mannheim, Deutschland) kloniert. Der resultierende Vektor pHWS 88 (DSM 8824) enthält das modifizierte Isomerasegen mit einer vorangestellten EcoRI-Restriktionsstelle vor dem ATG-Startkodon und den 3'-Bereich des Isomerasegens bis zur S1-verkürzten BstE II-Schnittstelle. Dieser Vektor verleiht unter Induktion mit IPTG den dieses Plasmid tragenden Zellen die Fähigkeit zur Isomeraseproduktion sowie die Resistenz gegen Ampicillin (50 bis 100 µg/ml). Vorzugsweise werden zur Erzeugung von Isomerase E.coli-Wirtszellen verwendet, die den lac-Repressor überproduzieren.

### 2. Herstellung des Plasmids pHWS118::Tn1721Tet

Die Genkasette für die Saccharose-Mutase wurde in ein Transposon eingebaut.

Dies geschah durch Einklonierung eines SphI/HindIII-DNA-Fragments aus dem Plasmid pHWS88, welches das Saccharose-MutaseGen unter Kontrolle des tac-Promotors trägt, in das Plasmid pJOE105, auf dem sich das Transposon Tn 1721 befindet. Das Plasmid pJOE105 wurde am 16. Dezember 1993 bei DSM unter der Hinterlegungsnummer DSM 8825 gemäß den Vorschriften des Budapester Vertrages hinterlegt. Das resultierende Plasmid pHWS118, auf dem sich das Saccharose-Mutasegen unter Kontrolle des regulierbaren tac-Promotors befindet, wurde verwendet, um einen F'-Plasmidhaltigen E.coli-Stamm zu transformieren. Fig. 2 zeigt das Klonierungsschema zur Herstellung von pHWS 118 aus pHWS88 und pJOE 105.

Die Herstellung von E.coli Transkonjuganten, die das Saccharose-Mutasegen enthalten, erfolgte nach dem in Fig. 3 beschriebenen Schema. Hierzu wurde als erstes der F'-tragende E.coli-Stamm CSH36 (J.H. Miller, Experiments in Molecular Genetics, Cold Spring Harbor Laboratory (1972), S. 18), der den durch das F'-Plasmid vermittelten Phänotyp Lac+ trägt, mit dem Nalidixinsäure-resistenten E.coli-Stamm JM108 (Sambrook et al., supra, S. A9-A13) gekreuzt. Durch Selektion auf Minimalmedium, dem Laktose, Prolin und Nalidixinsäure zugesetzt wurde, wurde ein F'-Lac-tragender Transkonjugant erhalten. Dieser wurde zusätzlich mit dem Iq-Plasmid FDX500 (Brinkmann et al., Gene 85 (1989), 109-114) transformiert, um eine Kontrolle des Saccharose-Mutase-Gens durch den tac-Promotor zu ermöglichen.

Der so vorbereitete Transkonjugant wurde mit dem Saccharose-Mutase-Gen tragenden Transposonplasmid pHWS118 transformiert. Zur Selektion von Transkonjuganten wurde in den Streptomycinresistenten E.coli-Stamm HB101 (Boyer und Roulland-Dussoix, J.Mol.Biol 41 (1969), 459-472) gekreuzt. Der Transfer der durch das Transposon vermittelten Tetracyclin-Resistenz war nur möglich nach Transposition des modifizierten Tn1721Tet von dem nicht konjugierbaren und nicht mobilisierbaren Plasmid pHWS118 auf das konjugierbare F'-Plasmid. Die Übertragung des F'-Plasmids mit dem modifizierten Transposon in HB101 wurde auf LB-Platten mit Streptomycin und Tetracyclin selektioniert und auf Ampicillin- und Nalidixinsäure-Platten nachgetestet.

### 3. Expression der Saccharose-Isomerase in E.coli

Untersuchungen zur Enzymproduktion solcher F'-Plasmid-tragenden E.coli-Zellen zeigten, daß Saccharose-Mutase-Protein produziert werden konnte. F'-Plasmid-haltige HB101-Zellen, die kein zusätzliches Lac-Repressor-Plasmid trugen (z.B. K1/1 oder K1/10), produzierten Saccharose-Mutase-Protein mit und ohne den Induktor Isopropyl-β-D-thiogalactosid (IPTG) in gleichen Mengen. Die Produktivitäten von drei Transkonjuganten K1/1, K1/10 und K1/4 sind in der folgenden Tabelle 1 wiedergegeben.

**Tabelle 1**

| Saccharose Mutase Aktivität in E.coli HB101 (F' ::Tn1721 [Mutase]) | | |
|---|---|---|
| Stamm | U/mg Mutase nach 4 Stunden ohne Induktion | U/mg Mutase nach 4 Stunden Induktion mit 50 µM IPTG |
| K1/1 | 1,0 | 1,2 |
| K1/10 | 0,9 | 1,1 |
| K1/4 | 0 | 1,6 |

Während der Produktion an Saccharose-Mutase-Protein konnte ein normales Wachstum der E.coli-Zellen beobachtet werden.

Erfolgte das Einbringen des Saccharose-Mutase-Gens in das F'-Plasmid in Gegenwart des Repressor-codierenden Plasmids pFDX500 (vgl. Transkonjugante K1/4), so wurde die Enzymproduktion durch den Induktor IPTG kontrollierbar. Während ohne IPTG keine enzymatische Aktivität gemessen wurde, konnte nach 4 Stunden Induktion eine Produktion des Saccharose-Mutase-Proteins von ca. 1,6 U/mg erhalten werden.

Eine Wachstumsbeeinträchtigung der Zellen konnte nicht beobachtet werden. Nach 4-stündiger Induktion erreichten die plasmidtragenden E.coli-Zellen eine Dichte von ca. 3 OD₆₀₀.

Es wurden bis zu 1,6 U/mg Saccharose-Mutase-Aktivität in transformierten E.coli gemessen. Die Syntheseleistung ist mit der von P. rubrum vergleichbar. Die Analyse des produzierten Enzyms durch SDS-Gelelektrophorese gibt keinen Hinweis auf inaktive Proteinaggregate. Die Bande des Saccharose-Mutase-Proteins war mit Coomassie-Färbung nur schwach sichtbar und nur deutlich im Westernblot detektierbar. Die Stärke der Proteinbande und gemessene enzymatische Aktivität waren bei der Produktion der Saccharose-Mutase in E.coli korrelierbar.

### BEISPIEL 3

### Isolierung des Saccharose-Isomerase-Gens aus Erwinia rhapontici

Es wurde auf gleiche Weise wie in Beispiel 1 beschrieben durch Restriktionsspaltung von Gesamt-DNA aus Erwinia rhapontici (NCPPB 1578) eine Genbank erzeugt.

Unter Verwendung der Primergemische 
5'-TGGTGGAAAGAAGCTGT-3'
           G G
 und 
5'-TCCCAGTTCAGGTCCGGCTG-3'
A
 wird durch eine PCR-Amplifikation ein DNA-Fragment erhalten, mit dessen Hilfe das Mutasegen enthaltende Kolonien durch Hybridisierung identifiziert werden können.

Auf diese Weise wurde ein positiver Klon pSST2023 gefunden, der ein 1305 Nukleotide langes Fragment des Erwinia-Isomerase-Gens enthält. Die Nukleotidsequenz dieses Fragments ist in SEQ ID NO. 2 dargestellt.

Im Sequenzvergleich mit dem Protaminobacter-Gen ergibt sich für den gesamten Genabschnitt unter Einbeziehung des Signalpeptidbereichs eine Identität von 77,7 % und eine Ähnlichkeit von 78 %, auf Aminosäure-Ebene eine Identität von 83,4 % bzw. eine Ähnlichkeit von 90,3 %.

Die Sequenzunterschiede konzentrieren sich hauptsächlich auf den Signalpeptidbereich. Deshalb sollte zum Vergleich ausschließlich der für die eigentliche Mutase-Aktivität verantwortliche Enzym-codierende Bereich, ohne Signalpeptid, betrachtet werden. Unter diesen Gesichtspunkten ergibt sich auf Nukleotid-Ebene eine Identität bzw. Ähnlichkeit von 79 %. Der Aminosäure-Sequenzvergleich (Fig. 1) in diesem Abschnitt weist 87,9 % identische Aminosäuren auf. Von 398 Aminosäuren (dies entspricht 71 % des gesamten Enzyms) der Erwinia-Mutase sind 349 gleich wie bei Protaminobacter. Von 48 ausgetauschten Aminosäuren weisen 25 eine starke Ähnlichkeit auf, so daß sich insgesamt auf AS-Ebene eine Ähnlichkeit von 94 % ergibt. Die AS-Austausche konzentrieren sich hauptsächlich auf die Region zwischen Aminosäure 141 und 198. Vor diesem Bereich liegt eine Folge von 56 konservierten Aminosäuren. Auch andere Abschnitte weisen eine besonders hohe Konservierung auf (vgl. Fig. 1).

Diese Daten zeigen, daß für den bisher klonierten und sequenzierten Abschnitt insgesamt eine sehr starke Konservierung der beiden Mutasen aus Erwinia und Protaminobacter gegeben ist.

### Identität des klonierten Mutase-Gens aus Erwinia

Für ein Rehybridisierungsexperiment mit genomischer Erwinia-DNA wurde als Sonde das ca. 500 bp große SspI/EcoRI-Fragment aus pSST2023 ausgewählt. Dieses Fragment wurde nach Digoxigenin-Markierung zur Hybridisierung mit Erwinia-DNA bei hoher Stringenz (68°C) eingesetzt. Mit SspI/EcoRI-geschnittener Erwinia-Gesamt-DNA ergab sich ein eindeutiges Hybridisierungssignal in erwarteter Größe von ca. 500 bp. Erwinia-DNA, welche ausschließlich mit SspI geschnitten wurde, ergab ein Hybridisierungssignal von ca. 2 kb.

Durch die erfolgreiche Rehybridisierung von pSST2023 mit genomischer Erwinia-DNA konnte abgesichert werden, daß der in pSST2023 klonierte Mutase-Bereich aus Erwinia rhapontici entstammt.

### Klonierung des C-terminalen Teilfragments der Erwinia-Mutase

Das bisher klonierte N-terminale Teilfragment des Erwinia-Mutase-Gens besitzt eine Größe von 1,3 kb und weist die in SEQ ID NO.2 gezeigte Nukleotidsequenz auf. Da für das gesamte Erwinia-Gen von einer nahezu identischen Größe wie für das bekannte Protaminobacter-Gen (1,8 kb) ausgegangen werden kann, fehlt im C-terminalen Bereich des Erwinia-Gens ein ca. 500 bp-Abschnitt.

Für die Klonierung des Erwinia-C-Terminus wurde das ca. 2 kb große SspI-Fragment aus Erwinia-Gesamt-DNA ausgewählt. Im SouthernBlot liefert dieses Fragment mit einer Digoxigenin-markierten DNA-Sonde aus pSST2023 ein eindeutiges Signal. Dieses 2 kb-SspI-Fragment überschneidet sich mit dem in pSST2023 bereits klonierten Bereich am 3'-Ende um ca. 500 bp. Seine Größe sollte zur vollständigen Klonierung des fehlenden Genabschnittes von ca. 500 bp ausreichend sein. Zur Identifikation gesuchter Klone eignet sich die Digoxigenin-markierte Fragment-Sonde SspI/EcoRI aus pSST2023.

### BEISPIEL 4

### Herstellung einer Protaminobacter Palatinase Defektmutante

Zellen von Protaminobacter rubrum (CBS 547, 77) wurden entsprechend der Methode von Adelberg et al. (Biochem. Biophys. Research Commun. 18 (1965), 788) modifiziert nach Miller, J., Experiments in Molecular Genetics, Cold Spring Harbor Laboratory, 125-179 (1972)) mit N-Methyl-N'-nitro-N-nitrosoguanidin mutagenisiert. Zur Selektion von Palatinase-Defektmutanten wurden MacConkey-Palatinose-Medium (MacConkey-Agar-Base (Difco Laboratories, Detroid, Michigan, USA), 40 g/l unter Zusatz von 20 g/l Palatinose, sterilfiltriert, 25 mg/l Kanamycin) und Minimalsalzmedien (10,5 g K₂HPO₄, 4,5 g KH₂PO₄, 1 g (NH₄)₂SO₄, 0, 5 g Natriumcitrat 2 H₂O, 0,1 g MgSO₄·7H₂O, 1 mg Thiamin, 2 g Palatinose oder Glucose, 25 mg Kanamycin und 15 g Agar pro Liter, pH 7,2) verwendet. Mutanten von P. rubrum, die auf MacConkey Palatinose-Medium weiß sind oder auf Minimalsalzmedium mit Glucose im Gegensatz zum gleichen Medium mit Palatinose wachsen, werden als Palatinase-Defekt-Mutanten identifiziert. In Zellextrakten aus den Mutanten kann die Enzymaktivität, Palatinose in Glucose und Fructose zu spalten (Palatinase-Aktivität), im Gegensatz zum Wildtyp nicht nachgewiesen werden. Züchtet man diese Zellen in Minimalsalzmedium mit 0,2 % Saccharose als einziger C-Quelle an, so ist im Gegensatz zu den Wildtypzellen, bei denen Palatinose nur transient in der Zeit von 4 bis 11 Stunden nach Kulturbeginn nachgewiesen werden kann, eine dauerhafte Akkumulation der Palatinose (Isomaltulose) nachweisbar. Übernachtkulturen in gleichem Medium enthalten im Falle der Wildtypzellen keine Palatinose, im Falle der auf diese Weise hergestellten Mutante SZZ 13 (DSM 9121) jedoch > 0,08 % Palatinose (siehe Fig. 4).

### BEISPIEL 5

### Immobilisierung von Mikroorganismen-Zellen

Von einer Abimpfung des entsprechenden Stammes werden Zellen mit 10 ml eines sterilen Nährsubstrats, bestehend aus 8 kg Dicksaft aus einer Zuckerfabrik (Trockensubstanzgehalt = 65 %), 2 kg Maisquellwasser, 0,1 kg (NH₄)₂HPO₄ und 89,9 kg destilliertes Wasser, pH 7,2, abgeschwemmt. Diese Suspension dient als Impfgut für die Schüttelmaschinen-Vorkultur in 1 1-Kolben mit 200 ml Nährlösung der obigen Zusammensetzung. Nach einer 30-stündigen Inkubationszeit bei 29°C werden mit 10 Kolben (Gesamtinhalt 2 1) 18 1 Nährlösung der obigen Zusammensetzung in einem 30 l Kleinfermenter beimpft und bei 29°C unter Zufuhr von 20 1 Luft pro Minute und einer Rührgeschwindigkeit von 350 Upm fermentiert.

Nach Erreichen von Keimzahlen über 5 x 10⁹ Keimen pro ml wird die Fermentation abgestellt und die Zellen durch Zentrifugation aus der Fermenterlösung abgeerntet. Die Zellen werden dann in eine 2 %ige Natriumalginatlösung suspendiert und durch Hineintropfen der Suspension in eine 2 %ige Calciumchloridlösung immobilisiert. Die entstandenen Immobilisatkugeln werden mit Wasser gewaschen und sind bei +4°C mehrere Wochen lagerfähig.

Zellen der Palatinase-Defektmutante SZZ 13 (DSM 9121) zeigen bessere katalytische Eigenschaften bezüglich ihrer Produktzusammensetzung als vergleichbare Zellen aus den bekannten Mikroorganismen Protaminobacter rubrum (CBS 547,77) und Erwinia rhapontici (NCPPB 1578).

Es wurden Ganzzellen und Rohextrakte von SZZ 13 sowie ein wie oben hergestelltes Immobilisat von SZZ 13 in Calciumalginat hinsichtlich seiner Produktzusammensetzung im Aktivitätstest bewertet. Vor dem eigentlichen Aktivitätstest wurde das Immobilisat in 0,1 mol/l Kaliumphosphatpuffer, pH 6,5 vorgequollen.

Die Aktivitätsmessungen bei 25°C ergaben, daß bei der Mutante SZZ 13 keine Fructose und Glucose gefunden wurden, während bei P. rubrum-Wildtypzellen in Ganzzellen 2,6 % und im Rohextrakt 12,0 % Fructose und Glucose (auf Basis der gesamten Mono- und Disaccharide) gefunden wurden. Bei E. rhapontici wurden in Ganzzellen 4 % und im Rohextrakt 41 % Glucose und Fructose gefunden.

### BEISPIEL 6

Isolierung des Saccharose-Isomerase-Gens aus anderen Mikroorganismen

Durch partiellen Verdau von genomischer DNA aus dem Isolat SZ62 (Enterobacter spec.), dem Organismus Pseudomonas mesoacidophila (MX-45) oder aus einem anderen Mikroorganismus und Einbringen der erhaltenen Fragmente in geeignete E.coli-Vektoren und Transformation wird eine Genbank gewonnen, deren Klone genomische Abschnitte zwischen 2 und 15 kb des Spenderorganismus enthalten.

Aus E.coli-Zellen, die diese Plasmide tragen, werden durch Plattierung auf McConkey-Palatinosemedium solche ausgewählt, die eine Rotfärbung der Kolonie aufweisen. Die in diesen Zellen enthaltene Plasmid-DNA wird in eine E.coli-Mutante überführt, die auf Galactose als einziger C-Quelle nicht wachsen kann (z.B. ED 8654, Sambrook et al., supra, Seiten A9-A13). Diese transformierte Zellinie ist zur Identifikation von Palatinoseproduzenten in der wie oben beschrieben hergestellten Genbank aus DNA des Spenderorganismus in der Lage.

Zur Identifikation der gesuchten Palatinose-bildenden Klone werden die Zellen der Genkbank auf Minimalsalzmedien mit Galactose und Saccharose vereinzelt und angezogen. Nach Replika-Stempeln der Kolonien auf Platten mit dem gleichen Medium werden die Zellen durch Bedampfung mit Toluol abgetötet. Anschließend werden Zellen des Screeningstamms als Rasen in Minimalsalz-Weichagar ohne C-Quellenzusatz über die Kolonien der Genbank ausgebracht und bebrütet. Es entsteht signifikantes Wachstum der Zellen des Screeningstamms nur am Ort von Zellen der Genbank, die Palatinose produziert haben. Bei Prüfung der Zellen der Replikakontrolle ergibt sich der Gehalt an Isomerase.

Diese so identifizierten E.coli-Klone sind auf Palatinose als einziger C-Quelle im Medium nicht wachstumsfähig, zeigen im Test der ganzen Zellen oder in Zellextrakten keine Fähigkeit zur Spaltung von Saccharose, bilden aber unter diesen Bedingungen und ohne Zusatz von Saccharose zum Medium bei der Anzucht Palatinose.

Alternativ können Isomerase-Klone auch unter Verwendung eines gemäß der Prozedur von Beispiel 3 hergestellten PCR-Fragments identifiziert werden.

Verwendet man Plasmid-DNA aus den so identifizierten E.coli--Klonen als Sonden zur Hybridisierung an Filtern mit immobilisierter DNA aus dem Spenderorganismus, lassen sich die Genbereiche, die Isomerasegene tragen, nachweisen und gezielt verfügbar machen.

Auf diese Weise wurde ein Klon identifiziert, der die in SEQ ID NO. 3 gezeigte Nukleotidsequenz mit der davon abgeleiteten und in SEQ ID NO. 6 gezeigten Aminosäuresequenz enthält. Ebenso wurde ein Isomerase-Klon aus DNA des Bakterienstamms Pseudomonas mesoacidophila MX-45 (FERM 11808) gefunden.

Die vollständige Nucleotid- und Aminosäuresequenz der Saccharose-Isomerase aus SZ 62 sind in SEQ ID NO. 11 und 12 dargestellt. Ein Großteil der Nukleotid- und Aminosäuresequenz der Saccharose-Isomerase aus MX-45 sind in SEQ ID NO. 13 und 14 dargestellt.

### BEISPIEL 7

### Klonierung eines Palatinase-Gens

Die in Beispiel 1 hergestellte Protaminobacter rubrum-Genbank wurde mit dem aus der Sequenz des N-Terminus der isolierten Palatinase abgeleiteten radioaktiv markierten Oligonukleotidgemisch S433 mit der Sequenz CA(G,A)TT(C,T)GG(T,C)TA(C,T)GG-3' gemustert.

Es wurde ein positiver Klon gefunden, aus dem ein Plasmid mit der Bezeichnung pKAT 203 isoliert wurde.

E.coli-Zellen, die das Plasmid pKAT203 tragen, sind zur Verstoffwechslung von Palatinose befähigt. Die im Aktivitätstest nachweisbare Spaltung von Palatinose in Glukose und Fructose läßt eine "Palatinase" vermuten.

Durch Sequenzierung von pKAT203-DNA mit dem Oligonukleotid S433 als Primer kann eine DNA-Sequenz erhalten werden, aus der nach Übersetzung in Aminosäuresequenzdaten die uns bekannten N-terminalen Aminosäuren abzulesen waren. Durch einen anschließenden sequenzierungsschritt wurde ein offener Leserahmen erhalten.

### Sequenzbestimmung des "Palatinase"-Gens

Zur weiteren Sequenzierung des "Palatinase"-Gens wurden Teilfragmente aus dem Plasmid pKAT 203 nach der Restriktionskarte ausgewählt, im M13-Phagensystem subkloniert und eine Sequenzierung der einzelsträngigen Phagen-DNA mit 'universal primer' 5'-GTTTTCCCAGTCACGAC-3' durchgeführt.

Werden die erhaltenen DNA-Sequenzdaten der einzelnen Fragmente unter Berücksichtigung überlappender Bereiche kombiniert, kann man einen durchgehenden Leserahmen für die "Palatinase" von 1360 Basenpaaren ermitteln (SEQ ID NO. 7).

Die Übersetzung dieser DNA-Sequenz in Aminosäuredaten ergibt ein Protein mit 453 Aminosäuren (SEQ ID NO. 8) und einem daraus abzuleitenden Molekulargewicht von ca. 50000 Da. Dies steht mit dem Befund in Einklang, daß durch Anreicherung der "Palatinase"-Aktivität eine Proteinfraktion erhalten werden konnte, die im SDS-Gel eine Bande bei ca. 48000 Da aufwies.
Im nativen Gel konnte die palatinosespaltende Aktivität einer Bande der Größe von ca. 150000 Da zugeschrieben werden.

Homologievergleiche mit anderen bekannten Proteinen

Durch Vergleich der aus der DNA-Sequenz ableitbaren Aminosäureabfolge mit Daten, die in einer Genbank (SwissProt) gespeichert sind, war eine Homologie zu Melibiase aus E.coli (MelA) auffallend (in zwei Teilen: identity 32 %).

### BEISPIEL 8

### Klonierung eines Palatinose-Hydrolase-Gens aus P. mesoacidophila MX-45

Aus der in Beispiel 6 hergestellten Genbank des Mikroorganismus P. mesoacidophila MX-45 wurde ein Gen mit der in SEQ ID NO. 15 gezeigten Nucleotidsequenz isoliert. Dieses Gen codiert für ein Protein mit der in SEQ ID NO. 16 gezeigten Aminosäuresequenz. Das Protein ist eine Palatinose-Hydrolase, welche die Spaltung von Palatinose unter Bildung von Fructose und Glucose katalysiert.

### SEQ ID NO. 1

NAME: Pr Isomerase LÄNGE: 1890 Basen BESCHREIBUNG Protaminobacter rubrum: Isomerase

*** S E Q U E N Z ***

1 ATGCCCCGTC AAGGATTGAA AACTGCACTA GCGATTTTTC TAACCACATC ATTATGCATC
61 TCATGCCAGC AAGCCTTTGG TACGCAACAA CCCTTGCTTA ACGAAAAGAG TATCGAACAG
121 TCGAAAACCA TACCTAAATG GTGGAAGGAG GCTGTTTTTT ATCAGGTGTA TCCGCGCTCC
181 TTTAAAGACA CCAACGGAGA TGGCATCGGG GATATTAACG GCATCATAGA AAAATTAGAC
241 TATCTAAAAG CCTTGGGGAT TGATGCCATT TGGATCAACC CACATTATGA TTCTCCGAAC
301 ACGGATAATG GTTACGATAT ACGTGATTAT CGAAAAATCA TGAAAGAATA TGGCACGATG
361 GAGGATTTTG ACCGCCTGAT TTCTGAAATG AAAAAACGGA ATATGCGGTT GATGATTGAT
421 GTGGTCATCA ACCACACCAG CGATCAAAAC GAATGGTTTG TTAAAAGTAA AAGCAGTAAG
481 GATAATCCTT ATCGCGGCTA TTATTTCTGG AAAGATGCTA AAGAAGGGCA GGCGCCTAAT
541 AATTACCCTT CATTCTTTGG TGGCTCGGCG TGGCAAAAAG ATGAAAAGAC CAATCAATAC
601 TACCTGCACT ATTTTGCTAA ACAACAGCCT GACCTAAACT GGGATAATCC CAAAGTCCGT
661 CAAGATCTTT ATGCAATGTT ACGTTTCTGG TTAGATAAAG GCGTGTCTGG TTTACGTTTT
721 GATACGGTAG CGACCTACTC AAAAATTCCG GATTTCCCAA ATCTCACCCA ACAACAGCTG
781 AAGAATTTTG CAGCGGAGTA TACCAAGGGC CCTAATATTC ATCGTTACGT CAATGAAATG
841 AATAAAGAGC TCTTGTCTCA TTACGACATT GCGACTGCCG GTGAAATCTT TGGCGTACCC
901 TTGGATCAAT CGATAAAGTT CTTCGATCGC CGCCGTGATG AGCTGAACAT TGCATTTACC
961 TTTGACTTAA TCAGACTCGA TCGAGACTCT GATCAAAGAT GGCGTCGAAA AGATTGGAAA
1021 TTGTCGCAAT TCCGGCAGAT CATCGATAAC GTTGACCGTA CTGCAGGAGA ATATGGTTGG
1081 AATGCCTTCT TCTTGGATAA CCACGACAAT CCGCGCGCTG TCTCGCACTT TGGCGATGAT
1141 GATCGCCCAC AATGGCGTGA GCCATCGGCT AAAGCGCTTG CAACCTTGAC GCTGACTCAA
1201 CGAGCAACAC CTTTTATTTA TCAAGGTTCA GAATTGGGCA TGACCAATTA CCCGTTTAAA
1261 GCTATTGATG AATTCGATGA TATTGAGGTG AAAGGTTTTT GGCATGACTA CGTTGAGACA
1321 GGAAAGGTCA AAGCCGACGA GTTCTTGCAA AATGTACGCC TGACGAGCAG GGATAACAGC
1381 CGGACGCCGT TCCAATGGGA TGGGAGCAAA AATGCAGGAT TCACGAGCGG AAAACCTTGG
1441 TTCAAGGTCA ACCCAAACTA CCAGGAAATC AATGCAGTAA GTCAAGTCAC ACAACCCGAC
1501 TCAGTATTTA ACTATTATCG TCAGTTGATC AAGATAAGGC ATGACATCCC GGCACTGACC
1561 TATGGTACAT ACACCGATTT GGATCCTGCA AATGATTCGG TCTACGCCTA TACACGCAGC
1621 CTTGGGGCGG AAAAATATCT TGTTGTTGTT AACTTCAAGG AGCAAATGAT GAGATATAAA

1681 TTACCGGATA ATTTATCCAT TGAGAAAGTG ATTATAGACA GCAACAGCAA AAACGTGGTG
1741 AAAAAGAATG ATTCATTACT CGAGCTAAAA CCATGGCAGT CAGGGGTTTA TAAAACTAAA
1801 TCAATAAATC TCATAGTCAC GCCAAATAAT GTAAATATAT TGAAACTATT AAAACCGGCA
1861 TTTTATGCCG GTTTTTTTAG CGCAAAATAG

### SEQ ID NO. 2

BESCHREIBUNG: E. rhapontici Isomerase
LÄNGE: 1305 Basen

*** SEQUENZ ***

1 ATGTCCTCTC AAGGATTGAA AACGGCTNTC GCTATTTTTC TTGCAACCAC TTTTTCTGCC
61 ACATCCTATC AGGCCTGCAG TGCCNNNCCA GATACCGCCC CCTCACTCAC CGTTCAGCAA
121 TCAAATGCCC TGCCCACATG GTGGAAGCAG GCTGTTTTTT ATCAGGTATA TCCACGCTCA
181 TTTAAAGATA CGAATGGGGA TGGCATTGGG GATTTAAACG GTATTATTGA GAATTTAGAC
241 TATCTGAAGA AACTGGGTAT TGATGCGATT TGGATCAATC CACATTACGA TTCGCCGAAT
301 ACGGATAATG GTTATGACAT CCGGGATTAC CGTAAGATAA TGAAAGAATA CGGTACGATG
361 GAAGACTTTG ACCGTCTTAT TTCAGAAATG AAGAAACGCA ATATGCGTTT GATGATTGAT
421 ATTGTTATCA ACCACACCAG CGATCAGCAT GCCTGGTTTG TTCAGAGCAA ATCGGGTAAG
481 AACAACCCCT ACAGGGACTA TTACTTCTGG CGTGACGGTA AGGATGGCCA TGCCCCCAAT
541 AACTATCCCT CCTTCTTCGG TGGCTCAGCC TGGGAAAAAG ACGATAAATC AGGCCAGTAT
601 TACCTCCATT ACTTTGCCAA ACAGCAACCC GACCTCAACT GGGACAATCC CAAAGTCCGT
661 CAAGACCTGT ATGACATGCT CCGCTTCTGG TTAGATAAAG GCGTTTCTGG TTTACGCTTT
721 GATACCGTTG CCACCTACTC GAAAATCCCG AACTTCCCTG ACCTTAGCCA ACAGCAGTTA
781 AAAAATTTCG CCGAGGAATA TACTAAAGGT CCTAAAATTC ACGACTACGT GAATGAAATG
841 AACAGAGAAG TATTATCCCA CTATGATATC GCCACTGCGG GGGAAATATT TGGGGTTCCT
901 CTGGATAAAT CGATTAAGTT TTTCGATCGC CGTAGAAATG AATTAAATAT AGCGTTTACG
961 TTTGATCTGA TCAGGCTCGA TCGTGATGCT GATGAAAGAT GGCGGCGAAA AGACTGGACC
1021 CTTTCGCAGT TCCGAAAAAT TGTCGATAAG GTTGACCAAA CGGCAGGAGA GTATGGGTGG
1081 AATGCCTTTT TCTTAGACAA TCACGACAAT CCCCGCGCGG TTTCTCACTT TGGTGATGAT
1141 CGACCACAAT GGCGCGAGCA TGCGGCGAAA GCACTGGCAA CATTGACGCT GACCCAGCGT
1201 GCAACGCCGT TTATCTATCA GGGTTCAGAA CTCGGTATGA CCAATTATCC CTTTAAAAAA
1261 ATCGATGATT TCGATGATGT AGAGGTGAAA GGTTTTTGGC AAGAC

### SEQ ID NO. 3

### NAME: SZISO1.DNA

BESCHREIBUNG: SZ62-Isomerase
LÄNGE: 471 Basen

*** SEQUENZ ***

1 GTTTTTTATC AGATCTATCC TCGCTCATTT AAAGACACCA ATGATGATGG CATTGGCGAT
61 ATTCGCGGTA TTATTGAAAA GCTGGACTAT CTGAAATCGC TCGGTATTGA CGCTATCTGG
121 ATCAATCCCC ATTACGACTC TCCGAACACC GATAACGGCT ATGACATCAG TAATTATCGT
181 CAGATAATGA AAGAGTATGG CACAATGGAG GATTTTGATA GCCTTGTTGC CGAAATGAAA
241 AAACGAAATA TGCGCTTAAT GATCGACGTG GTCATTAACC ATACCAGTGA TCAACACCCG
301 TGGTTTATTC AGAGTAAAAG CGATAAAAAC AACCCTTATC GTGACTATTA TTTCTGGCGT
361 GACGGAAAAG ATAATCAGCC ACCTAATAAT TACCCCTCAT TTTTCGGCGG CTCGGCATGG
421 CAAAAAGATG CAAAGTCAGG ACAGTACTAT TTACACTATT TTGCCAGACA G

### SEQ ID NO. 4

NAME: Pr Isomerase LÄNGE: 1890 Basen oder 629 Aminosäuren BESCHREIBUNG: Protaminobacter rubrum: Isomerase

*** S E Q U E N Z ***

1 ATG CCC CGT CAA GGA TTG AAA ACT GCA CTA GCG ATT TTT CTA ACC ACA
Met Pro Arg Gln Gly Leu Lys Thr Ala Leu Ala Ile Phe Leu Thr Thr

49 TCA TTA TGC ATC TCA TGC CAG CAA GCC TTC GGT ACG CAA CAA CCC TTG
Ser Leu Cys Ile Ser Cys Gln Gln Ala Phe Gly Thr Gln Gln Pro Leu

97 CTT AAC GAA AAG AGT ATC GAA CAG TCG AAA ACC ATA CCT AAA TGG TGG
Leu Asn Glu Lys Ser Ile Glu Gln Ser Lys Thr Ile Pro Lys Trp Trp

145 AAG GAG GCT GTT TTT TAT CAG GTG TAT CCG CGC TCC TTT AAA GAC ACC
Lys Glu Ala Val Phe Tyr Gln Val Tyr Pro Arg Ser Phe Lys Asp Thr

193 AAC GGA GAT GGC ATC GGG GAT ATT AAC GGC ATC ATA GAA AAA TTA GAC
Asn Gly Asp Gly Ile Gly Asp Ile Asn Gly Ile Ile Glu Lys Leu Asp

241 TAT CTA AAA GCC TTG GGG ATT GAT GCC ATT TGG ATC AAC CCA CAT TAT
Tyr Leu Lys Ala Leu Gly Ile Asp Ala Ile Trp Ile Asn Pro His Tyr

289 GAT TCT CCG AAC ACG GAT AAT GGT TAC GAT ATA CGT GAT TAT CGA AAA
Asp Ser Pro Asn Thr Asp Asn Gly Tyr Asp Ile Arg Asp Tyr Arg Lys

337 ATC ATG AAA GAA TAT GGC ACG ATG GAG GAT TTT GAC CGC CTG ATT TCT
Ile Met Lys Glu Tyr Gly Thr Met Glu Asp Phe Asp Arg Leu Ile Ser

385 GAA ATG AAA AAA CGG AAT ATG CGG TTG ATG ATT GAT GTG GTC ATC AAC
Glu Met Lys Lys Arg Asn Met Arg Leu Met Ile Asp Val Val Ile Asn

433 CAC ACC AGC GAT CAA AAC GAA TGG TTT GTT AAA AGT AAA AGC AGT AAG
His Thr Ser Asp Gln Asn Glu Trp Phe Val Lys Ser Lys Ser Ser Lys

481 GAT AAT CCT TAT CGC GGC TAT TAT TTC TGG AAA GAT GCT AAA GAA GGG
Asp Asn Pro Tyr Arg Gly Tyr Tyr Phe Trp Lys Asp Ala Lys Glu Gly

529 CAG GCG CCT AAT AAT TAC CCT TCA TTC TTT GGT GGC TCG GCG TGG CAA
Gln Ala Pro Asn Asn Tyr Pro Ser Phe Phe Gly Gly Ser Ala Trp Gln

577 AAA GAT GAA AAG ACC AAT CAA TAC TAC CTG CAC TAT TTT GCT AAA CAA
Lys Asp Glu Lys Thr Asn Gln Tyr Tyr Leu His Tyr Phe Ala Lys Gln

625 CAG CCT GAC CTA AAC TGG GAT AAT CCC AAA GTC CGT CAA GAT CTT TAT
Gln Pro Asp Leu Asn Trp Asp Asn Pro Lys Val Arg Gln Asp Leu Tyr

673 GCA ATG TTA CGT TTC TGG TTA GAT AAA GGC GTG TCT GGT TTA CGT TTT
Ala Met Leu Arg Phe Trp Leu Asp Lys Gly Val Ser Gly Leu Arg Phe

721 GAT ACG GTA GCG ACC TAC TCA AAA ATT CCG GAT TTC CCA AAT CTC ACC
Asp Thr Val Ala Thr Tyr Ser Lys Ile Pro Asp Phe Pro Asn Leu Thr

769 CAA CAA CAG CTG AAG AAT TTT GCA GCG GAG TAT ACC AAG GGC CCT AAT
Gln Gln Gln Leu Lys Asn Phe Ala Ala Glu Tyr Thr Lys Gly Pro Asn

817 ATT CAT CGT TAC GTC AAT GAA ATG AAT AAA GAG GTC TTG TCT CAT TAC
Ile His Arg Tyr Val Asn Glu Met Asn Lys Glu Val Leu Ser His Tyr

865 GAC ATT GCG ACT GCC GGT GAA ATC TTT GGC GTA CCC TTG GAT CAA TCG
Asp Ile Ala Thr Ala Gly Glu Ile Phe Gly Val Pro Leu Asp Gln Ser

913 ATA AAG TTC TTC GAT CGC CGC CGT GAT GAG CTG AAC ATT GCA TTT ACC
Ile Lys Phe Phe Asp Arg Arg Arg Asp Glu Leu Asn Ile Ala Phe Thr

961 TTT GAC TTA ATC AGA CTC GAT CGA GAC TCT GAT CAA AGA TGG CGT CGA
Phe Asp Leu Ile Arg Leu Asp Arg Asp Ser Asp Gln Arg Trp Arg Arg

1009 AAA GAT TGG AAA TTG TCG CAA TTC CGG CAG ATC ATC GAT AAC GTT GAC
Lys Asp Trp Lys Leu Ser Gln Phe Arg Gln Ile Ile Asp Asn Val Asp

1057 CGT ACT GCA GGA GAA TAT GGT TGG AAT GCC TTC TTC TTG GAT AAC CAC
Arg Thr Ala Gly Glu Tyr Gly Trp Asn Ala Phe Phe Leu Asp Asn His

1105 GAC AAT CCG CGC GCT GTC TCG CAC TTT GGC GAT GAT GAT CGC CCA CAA
Asp Asn Pro Arg Ala Val Ser His Phe Gly Asp Asp Asp Arg Pro Gln

1153 TGG CGT GAG CCA TCG GCT AAA GCG CTT GCA ACC TTG ACG CTG ACT CAA
Trp Arg Glu Pro Ser Ala Lys Ala Leu Ala Thr Leu Thr Leu Thr Gln

1201 CGA GCA ACA CCT TTT ATT TAT CAA GGT TCA GAA TTG GGC ATG ACC AAT
Arg Ala Thr Pro Phe Ile Tyr Gln Gly Ser Glu Leu Gly Met Thr Asn

1249 TAC CCG TTT AAA GCT ATT GAT GAA TTC GAT GAT ATT GAG GTG AAA GGT
Tyr Pro Phe Lys Ala Ile Asp Glu Phe Asp Asp Ile Glu Val Lys Gly

1297 TTT TGG CAT GAC TAC GTT GAG ACA GGA AAG GTC AAA GCC GAC GAG TTC
Phe Trp His Asp Tyr Val Glu Thr Gly Lys Val Lys Ala Asp Glu Phe

1345 TTG CAA AAT GTA CGC CTG ACG AGC AGG GAT AAC AGC CGG ACG CCG TTC
Leu Gln Asn Val Arg Leu Thr Ser Arg Asp Asn Ser Arg Thr Pro Phe

1393 CAA TGG GAT GGG AGC AAA AAT GCA GGA TTC ACG AGC GGA AAA CCT TGG
Gln Trp Asp Gly Ser Lys Asn Ala Gly Phe Thr Ser Gly Lys Pro Trp

1441 TTC AAG GTC AAC CCA AAC TAC CAG GAA ATC AAT GCA GTA AGT CAA GTC
Phe Lys Val Asn Pro Asn Tyr Gln Glu Ile Asn Ala Val Ser Gln Val

1489 ACA CAA CCC GAC TCA GTA TTT AAC TAT TAT CGT CAG TTG ATC AAG ATA
Thr Gln Pro Asp Ser Val Phe Asn Tyr Tyr Arg Gln Leu Ile Lys Ile

1537 AGG CAT GAC ATC CCG GCA CTG ACC TAT GGT ACA TAC ACC GAT TTG GAT
Arg His Asp Ile Pro Ala Leu Thr Tyr Gly Thr Tyr Thr Asp Leu Asp

1585 CCT GCA AAT GAT TCG GTC TAC GCC TAT ACA CGC AGC CTT GGG GCG GAA
Pro Ala Asn Asp Ser Val Tyr Ala Tyr Thr Arg Ser Leu Gly Ala Glu

1633 AAA TAT CTT GTT GTT GTT AAC TTC AAG GAG CAA ATG ATG AGA TAT AAA
Lys Tyr Leu Val Val Val Asn Phe Lys Glu Gln Met Met Arg Tyr Lys

1681 TTA CCG GAT AAT TTA TCC ATT GAG AAA GTG ATT ATA GAC AGC AAC AGC
Leu Pro Asp Asn Leu Ser Ile Glu Lys Val Ile Ile Asp Ser Asn Ser

1729 AAA AAC GTG GTG AAA AAG AAT GAT TCA TTA CTC GAG CTA AAA CCA TGG
Lys Asn Val Val Lys Lys Asn Asp Ser Leu Leu Glu Leu Lys Pro Trp

1777 CAG TCA GGG GTT TAT AAA ACT AAA TCA ATA AAT CTC ATA GTC ACG CCA
Gln Ser Gly Val Tyr Lys Thr Lys Ser Ile Asn Leu Ile Val Thr Pro

1825 AAT AAT GTA AAT ATA TTG AAA CTA TTA AAA CCG GCA TTT TAT GCC GGT
Asn Asn Val Asn Ile Leu Lys Leu Leu Lys Pro Ala Phe Tyr Ala Gly

1873 TTT TTT AGC GCA AAA TAG
Phe Phe Ser Ala Lys ***

### SEO ID NO. 5

BESCHREIBUNG: E. rhapontici Isomerase
LÄNGE: 1305 Basen oder 435 Aminosäuren

*** SEQUENZ ***

1 ATG TCC TCT CAA GGA TTG AAA ACG GCT NTC GCT ATT TTT CTT GCA ACC
Met Ser Ser Gln Gly Leu Lys Thr Ala ? Ala Ile Phe Leu Ala Thr

49 ACT TTT TCT GCC ACA TCC TAT CAG GCC TGC AGT GCC NNN CCA GAT ACC
Thr Phe Ser Ala Thr Ser Tyr Gln Ala Cys Ser Ala ? Pro Asp Thr

97 GCC CCC TCA CTC ACC GTT CAG CAA TCA AAT GCC CTG CCC ACA TGG TGG
Ala Pro Ser Leu Thr Val Gln Gln Ser Asn Ala Leu Pro Thr Trp Trp

145 AAG CAG GCT GTT TTT TAT CAG GTA TAT CCA CGC TCA TTT AAA GAT ACG
Lys Gln Ala Val Phe Tyr Gln Val Tyr Pro Arg Ser Phe Lys Asp Thr

193 AAT GGG GAT GGC ATT GGG GAT TTA AAC GGT ATT ATT GAG AAT TTA GAC
Asn Gly Asp Gly Ile Gly Asp Leu Asn Gly Ile Ile Glu Asn Leu Asp

241 TAT CTG AAG AAA CTG GGT ATT GAT GCG ATT TGG ATC AAT CCA CAT TAC
Tyr Leu Lys Lys Leu Gly Ile Asp Ala Ile Trp Ile Asn Pro His Tyr

289 GAT TCG CCG AAT ACG GAT AAT GGT TAT GAC ATC CGG GAT TAC CGT AAG
Asp Ser Pro Asn Thr Asp Asn Gly Tyr Asp Ile Arg Asp Tyr Arg Lys

337 ATA ATG AAA GAA TAC GGT ACG ATG GAA GAC TTT GAC CGT CTT ATT TCA
Ile Met Lys Glu Tyr Gly Thr Met Glu Asp Phe Asp Arg Leu Ile Ser

385 GAA ATG AAG AAA CGC AAT ATG CGT TTG ATG ATT GAT ATT GTT ATC AAC
Glu Met Lys Lys Arg Asn Met Arg Leu Met Ile Asp Ile Val Ile Asn

433 CAC ACC AGC GAT CAG CAT GCC TGG TTT GTT CAG AGC AAA TCG GGT AAG
His Thr Ser Asp Gln His Ala Trp Phe Val Gln Ser Lys Ser Gly Lys

481 AAC AAC CCC TAC AGG GAC TAT TAC TTC TGG CGT GAC GGT AAG GAT GGC
Asn Asn Pro Tyr Arg Asp Tyr Tyr Phe Trp Arg Asp Gly Lys Asp Gly

529 CAT GCC CCC AAT AAC TAT CCC TCC TTC TTC GGT GGC TCA GCC TGG GAA
His Ala Pro Asn Asn Tyr Pro Ser Phe Phe Gly Gly Ser Ala Trp Glu

577 AAA GAC GAT AAA TCA GGC CAG TAT TAC CTC CAT TAC TTT GCC AAA CAG
Lys Asp Asp Lys Ser Gly Gln Tyr Tyr Leu His Tyr Phe Ala Lys Gln

625 CAA CCC GAC CTC AAC TGG GAC AAT CCC AAA GTC CGT CAA GAC CTG TAT
Gln Pro Asp Leu Asn Trp Asp Asn Pro Lys Val Arg Gln Asp Leu Tyr

673 GAC ATG CTC CGC TTC TGG TTA GAT AAA GGC GTT TCT GGT TTA CGC TTT
Asp Met Leu Arg Phe Trp Leu Asp Lys Gly Val Ser Gly Leu Arg Phe

721 GAT ACC GTT GCC ACC TAC TCG AAA ATC CCG AAC TTC CCT GAC CTT AGC
Asp Thr Val Ala Thr Tyr Ser Lys Ile Pro Asn Phe Pro Asp Leu Ser

769 CAA CAG CAG TTA AAA AAT TTC GCC GAG GAA TAT ACT AAA GGT CCT AAA
Gln Gln Gln Leu Lys Asn Phe Ala Glu Glu Tyr Thr Lys Gly Pro Lys

817 ATT CAC GAC TAC GTG AAT GAA ATG AAC AGA GAA GTA TTA TCC CAC TAT
Ile His Asp Tyr Val Asn Glu Met Asn Arg Glu Val Leu Ser His Tyr

865 GAT ATC GCC ACT GCG GGG GAA ATA TTT GGG GTT CCT CTG GAT AAA TCG
Asp Ile Ala Thr Ala Gly Glu Ile Phe Gly Val Pro Leu Asp Lys Ser

913 ATT AAG TTT TTC GAT CGC CGT AGA AAT GAA TTA AAT ATA GCG TTT ACG
Ile Lys Phe Phe Asp Arg Arg Arg Asn Glu Leu Asn Ile Ala Phe Thr

961 TTT GAT CTG ATC AGG CTC GAT CGT GAT GCT GAT GAA AGA TGG CGG CGA
Phe Asp Leu Ile Arg Leu Asp Arg Asp Ala Asp Glu Arg Trp Arg Arg

1009 AAA GAC TGG ACC CTT TCG CAG TTC CGA AAA ATT GTC GAT AAG GTT GAC
Lys Asp Trp Thr Leu Ser Gln Phe Arg Lys Ile Val Asp Lys Val Asp

1057 CAA ACG GCA GGA GAG TAT GGG TGG AAT GCC TTT TTC TTA GAC AAT CAC
Gln Thr Ala Gly Glu Tyr Gly Trp Asn Ala Phe Phe Leu Asp Asn His

1105 GAC AAT CCC CGC GCG GTT TCT CAC TTT GGT GAT GAT CGA CCA CAA TGG
Asp Asn Pro Arg Ala Val Ser His Phe Gly Asp Asp Arg Pro Gln Trp

1153 CGC GAG CAT GCG GCG AAA GCA CTG GCA ACA TTG ACG CTG ACC CAG CGT
Arg Glu His Ala Ala Lys Ala Leu Ala Thr Leu Thr Leu Thr Gln Arg

1201 GCA ACG CCG TTT ATC TAT CAG GGT TCA GAA CTC GGT ATG ACC AAT TAT
Ala Thr Pro Phe Ile Tyr Gln Gly Ser Glu Leu Gly Met Thr Asn Tyr

1249 CCC TTT AAA AAA ATC GAT GAT TTC GAT GAT GTA GAG GTG AAA GGT TTT
Pro Phe Lys Lys Ile Asp Asp Phe Asp Asp Val Glu Val Lys Gly Phe

1297 TGG CAA GAC
Trp Gln Asp

### SEQ ID NO. 6

### NAME: SZISO1.DNA

BESCHREIBUNG: SZ62-Isomerase
LÄNGE: 471 Basen

*** SEQUENZ ***

1 GTT TTT TAT CAG ATC TAT CCT CGC TCA TTT AAA GAC ACC AAT GAT GAT
Val Phe Tyr Gln Ile Tyr Pro Arg Ser Phe Lys Asp Thr Asn Asp Asp

49 GGC ATT GGC GAT ATT CGC GGT ATT ATT GAA AAG CTG GAC TAT CTG AAA
Gly Ile Gly Asp Ile Arg Gly Ile Ile Glu Lys Leu Asp Tyr Leu Lys

97 TCG CTC GGT ATT GAC GCT ATC TGG ATC AAT CCC CAT TAC GAC TCT CCG
Ser Leu Gly Ile Asp Ala Ile Trp Ile Asn Pro His Tyr Asp Ser Pro

145 AAC ACC GAT AAC GGC TAT GAC ATC AGT AAT TAT CGT CAG ATA ATG AAA
Asn Thr Asp Asn Gly Tyr Asp Ile Ser Asn Tyr Arg Gln Ile Met Lys

193 GAG TAT GGC ACA ATG GAG GAT TTT GAT AGC CTT GTT GCC GAA ATG AAA
Glu Tyr Gly Thr Met Glu Asp Phe Asp Ser Leu Val Ala Glu Met Lys

241 AAA CGA AAT ATG CGC TTA ATG ATC GAC GTG GTC ATT AAC CAT ACC AGT
Lys Arg Asn Met Arg Leu Met Ile Asp Val Val Ile Asn His Thr Ser

289 GAT CAA CAC CCG TGG TTT ATT CAG AGT AAA AGC GAT AAA AAC AAC CCT
Asp Gln His Pro Trp Phe Ile Gln Ser Lys Ser Asp Lys Asn Asn Pro

337 TAT CGT GAC TAT TAT TTC TGG CGT GAC GGA AAA GAT AAT CAG CCA CCT
Tyr Arg Asp Tyr Tyr Phe Trp Arg Asp Gly Lys Asp Asn Gln Pro Pro

385 AAT AAT TAC CCC TCA TTT TTC GGC GGC TCG GCA TGG CAA AAA GAT GCA
Asn Asn Tyr Pro Ser Phe Phe Gly Gly Ser Ala Trp Gln Lys Asp Ala

433 AAG TCA GGA CAG TAC TAT TTA CAC TAT TTT GCC AGA CAG
Lys Ser Gly Gln Tyr Tyr Leu His Tyr Phe Ala Arg Gln

### SEQ ID NO. 7

NAME: PALA.SEQ LÄNGE: 1362 Basen
BESCHREIBUNG: Palatinase

*** S E Q U E N Z ***

ATGGCTACAA AAATCGTTTT AGTGGGCGCA GGCAGCGCGC AATTCGGCTA CGGCACCCTG
61 GGCGATATCT TCCAGAGCAA GACGCTGTAC GGCAGTGAAA TTGTGCTGCA TGACATCAAC
121 CCAACCTCGC TGGCGGTGAC CGAGAAAACC GCCCGTGACT TCCTGGCTGC GGAAGATCTG
181 CCGTTTATCG TCAGCGCCAC CACCGATCGC AAAACCGCGC TGAGCGGAGC GGAGTTCGTG
241 ATTATC CCA TTGA GTGGG CGACCGCTDT GCCCTGTGGG ATCTCGACTG GCAGATCCCG
301 CAACAGTATG GCATTCAGCA GGTGTATGGT GAAAACGGTG GCCCTGGCGG GCTGTTCCAC
361 TCGCTGCGCA TCATTCCACC GATCCTCGAC ATCTGCGCCG ACGTGGCGGA CATTTGCCCG
421 AACGCCTGGG TATTCAACTA CTCGAACCCG ATGAGCCGCA TTTGCACCAC CGTGCATCGC
481 CGTTTCCCGC AGCTCAACTT TGTCGGCATG TGCCATGAAA TCGCCTCACT TGAGCGTTAT
541 CTGCCAGAAA TGCTCGGCAC CTCCTTCGAC AATCTCACTC TGCGCGCTGC CGGGCTGAAC
601 CACTTCAGCG TGTTGCTGGA GGCCAGCTAT AAAGACAGCG GAAAAGACGC TTACGCCGAC
661 GTACGCGCCA AGGCACCGGA CTATTTCTCC CGTCTGCCGG GCTACAGCGA TATTCTGGCT
721 TACACCCGCA ATCACGGCAA ATTGGTGGAG ACAGAAGGCA GCACCGAACG CGATGCGCTG
781 GGCGGCAAAG ACAGCGCCTA TCCGTGGGCG GACCGCACGC TGTTCAAAGA GATCCTGGAG
841 AAGTTTCACC ATTTGCCGAT CACCGGCGAC AGCCACTTTG GCGAGTACAT CCGTTGGGCC
901 AGCGAAGTCA GCGATCACCG CGGTATCCTC GATTTCTACA CCTTCTACCG CAACTATCTG
961 GGGCATGTGC AGCCAAAAAT CGAACTGAAG CTGAAAGAAC GCGTGGTGCC GATCATGGAA
1021 GGGATCCTCA CCGATTCCGG TTATGAAGAG TCTGCGGTCA ACATTCCGAA CCAGGGATTT
1081 ATCAAGCAAC TGCCGGCGTT TATTGCCGTC GAAGTCCCGG CGATTATCGA CCGCAAGGGC
1141 GTGCACGGCA TCAAGGTCGA TATGCCTGCG GGCATCGGTG GCCTGTTGAG CAACCAGATT
1201 GCGATTCACG ATCTGACCGC CGACGCAGTG ATTGAAGGCT CGCGCGACCT GGTTATCCAG
1261 GCGCTGCTGG TGGACTCGGT CAACGATAAA TGCCGCGCGA TACCGGAACT GGTGGACGTG
1321 ATGATCTCAC GCCAGGGGCC GTGGCTCGAT TACCTGAAAT AA

### SEQ ID NO. 8

NAME: PALA.SEQ LÄNGE: 1362 Basen oder 453 Aminosäuren BESCHREIBUNG: Palatinase

*** SEQUENZ ***

1 ATG GCT ACA AAA ATC GTT TTA GTG GGC GCA GGC AGC GCG CAA TTC GGC
Met Ala Thr Lys Ile Val Leu Val Gly Ala Gly Ser Ala Gln Phe Gly

49 TAC GGC ACC CTG GGC GAT ATC TTC CAG AGC AAG ACG CTG TAC GGC AGT
Tyr Gly Thr Leu Gly Asp Ile Phe Gln Ser Lys Thr Leu Tyr Gly Ser

97 GAA ATT GTG CTG CAT GAC ATC AAC CCA ACC TCG CTG GCC GTG ACC GAG
Glu Ile Val Leu His Asp Ile Asn Pro Thr Ser Leu Ala Val Thr Glu

145 AAA ACC GCC CGT GAC TTC CTG GCT GCG GAA GAT CTG CCG TTT ATC GTC
Lys Thr Ala Arg Asp Phe Leu Ala Ala Glu Asp Leu Pro Phe Ile Val

193 AGC GCC ACC ACC GAT CGC AAA ACC GCG CTG AGC GGA GCG GAG TTC GTG
Ser Ala Thr Thr Asp Arg Lys Thr Ala Leu Ser Gly Ala Glu Phe Val

241 ATT ATC TCC ATT GAA GTG GGC GAC CGC TTT GCC CTG TGG GAT CTC GAC
Ile Ile Ser Ile Glu Val Gly Asp Arg Phe Ala Leu Trp Asp Leu Asp

289 TGG CAG ATC CCG CAA CAG TAT GGC ATT CAG CAG GTG TAT GGT GAA AAC
Trp Gln Ile Pro Gln Gln Tyr Gly Ile Gln Gln Val Tyr Gly Glu Asn

337 GGT GGC CCT GGC GGG CTG TTC CAC TCG CTG CGC ATC ATT CCA CCG ATC
Gly Gly Pro Gly Gly Leu Phe His Ser Leu Arg Ile Ile Pro Pro Ile

385 CTC GAC ATC TGC GCC GAC GTG GCG GAC ATT TGC CCG AAC GCC TGG GTA
Leu Asp Ile Cys Ala Asp Val Ala Asp Ile Cys Pro Asn Ala Trp Val

433 TTC AAC TAC TCG AAC CCG ATG AGC CGC ATT TGC ACC ACC GTG CAT CGC
Phe Asn Tyr Ser Asn Pro Met Ser Arg Ile Cys Thr Thr Val His Arg

481 CGT TTC CCG CAG CTC AAC TTT GTC GGC ATG TGC CAT GAA ATC GCC TCA
Arg Phe Pro Gln Leu Asn Phe Val Gly Met Cys His Glu Ile Ala Ser

529 CTT GAG CGT TAT CTG CCA GAA ATG CTC GGC ACC TCC TTC GAC AAT CTC
Leu Glu Arg Tyr Leu Pro Glu Met Leu Gly Thr Ser Phe Asp Asn Leu

577 ACT CTG CGC GCT GCC GGG CTG AAC CAC TTC AGC GTG TTG CTG GAG GCC
Thr Leu Arg Ala Ala Gly Leu Asn His Phe Ser Val Leu Leu Glu Ala

625 AGC TAT AAA GAC AGC GGA AAA GAC GCT TAC GCC GAC GTA CGC GCC AAG
Ser Tyr Lys Asp Ser Gly Lys Asp Ala Tyr Ala Asp Val Arg Ala Lys

673 GCA CCG GAC TAT TTC TCC CGT CTG CCG GGC TAC AGC GAT ATT CTG GCT
Ala Pro Asp Tyr Phe Ser Arg Leu Pro Gly Tyr Ser Asp Ile Leu Ala

721 TAC ACC CGC AAT CAC GGC AAA TTG GTG GAG ACA GAA GGC AGC ACC GAA
Tyr Thr Arg Asn His Gly Lys Leu Val Glu Thr Glu Gly Ser Thr Glu

769 CGC GAT GCG CTG GGC GGC AAA GAC AGC GCC TAT CCG TGG GCG GAC CGC
Arg Asp Ala Leu Gly Gly Lys Asp Ser Ala Tyr Pro Trp Ala Asp Arg

817 ACG CTG TTC AAA GAG ATC CTG GAG AAG TTT CAC CAT TTG CCG ATC ACC
Thr Leu Phe Lys Glu Ile Leu Glu Lys Phe His His Leu Pro Ile Thr

865 GGC GAC AGC CAC TTT GGC GAG TAC ATC CGT TGG GCC AGC GAA GTC AGC
Gly Asp Ser His Phe Gly Glu Tyr Ile Arg Trp Ala Ser Glu Val Ser

913 GAT CAC CGC GGT ATC CTC GAT TTC TAC ACC TTC TAC CGC AAC TAT CTG
Asp His Arg Gly Ile Leu Asp Phe Tyr Thr Phe Tyr Arg Asn Tyr Leu

961 GGG CAT GTG CAG CCA AAA ATC GAA CTG AAG CTG AAA GAA CGC GTG GTG
Gly His Val Gln Pro Lys Ile Glu Leu Lys Leu Lys Glu Arg Val Val

1009 CCG ATC ATG GAA GGG ATC CTC ACC GAT TCC GGT TAT GAA GAG TCT GCG
Pro Ile Met Glu Gly Ile Leu Thr Asp Ser Gly Tyr Glu Glu Ser Ala

1057 GTC AAC ATT CCG AAC CAG GGA TTT ATC AAG CAA CTG CCG GCG TTT ATT
Val Asn Ile Pro Asn Gln Gly Phe Ile Lys Gln Leu Pro Ala Phe Ile

1105 GCC GTC GAA GTC CCG GCG ATT ATC GAC CGC AAG GGC GTG CAC GGC ATC
Ala Val Glu Val Pro Ala Ile Ile Asp Arg Lys Gly Val His Gly Ile

1153 AAG GTC GAT ATG CCT GCG GGC ATC GGT GGC CTG TTG AGC AAC CAG ATT
Lys Val Asp Met Pro Ala Gly Ile Gly Gly Leu Leu Ser Asn Gln Ile

1201 GCG ATT CAC GAT CTG ACC GCC GAC GCA GTG ATT GAA GGC TCG CGC GAC
Ala Ile His Asp Leu Thr Ala Asp Ala Val Ile Glu Gly Ser Arg Asp

1249 CTG GTT ATC CAG GCG CTG CTG GTG GAC TCG GTC AAC GAT AAA TGC CGC
Leu Val Ile Gln Ala Leu Leu Val Asp Ser Val Asn Asp Lys Cys Arg

1297 GCG ATA CCG GAA CTG GTG GAC GTG ATG ATC TCA CGC CAG GGG CCG TGG
Ala Ile Pro Glu Leu Val Asp Val Met Ile Ser Arg Gln Gly Pro Trp

1345 CTC GAT TAC CTG AAA TAA
Leu Asp Tyr Leu Lys ---

### SEQ ID NO. 9

BESCHREIBUNG: P. rubrum Isomerase (Variante)
LÄNGE: 1803 Basenpaare

*** S E Q U E N Z ***

1 ATGCCCCGTC AAGGATTGAA AACTGCACTA GCGATTTTTC TAACCACATC ATTATGCATC
61 TCATGCCAGC AAGCCTTCGG TACGCAACAA CCCTTGCTTA ACGAAAAGAG TATCGAACAG
121 TCGAAAACCA TACCTAAATG GTGGAAGGAG GCTGTTTTTT ATCAGGTGTA TCCGCGCTCC
181 TTTAAAGACA CCAACGGAGA TGGCATCGGG GATATTAACG GCATCATAGA AAAATTAGAC
241 TATCTAAAAG CCTTGGGGAT TGATGCCATT TGGATCAACC CACATTATGA TTCTCCGAAC
301 ACGGATAATG GTTACGATAT ACGTGATTAT CGAAAAATCA TGAAAGAATA TGGCACGATG
361 GAGGATTTTG ACCGCCTGAT TTCTGAAATG AAAAAACGGA ATATGCGGTT GATGATTGAT
421 GTGGTCATCA ACCACACCAG CGATCAAAAC GAATGGTTTG TTAAAAGTAA AAGCAGTAAG
481 GATAATCCTT ATCGCGGCTA TTATTTCTGG AAAGATGCTA AAGAAGGGCA GGCGCCTAAT
541 AATTACCCTT CATTCTTTGG TGGCTCGGCG TGGCAAAAAG ATGAAAAGAC CAATCAATAC
601 TACCTGCACT ATTTTGCTAA ACAACAGCCT GACCTAAACT GGGATAATCC CAAAGTCCGT
661 CAAGATCTTT ATGCAATGTT ACGTTTCTGG TTAGATAAAG GCGTGTCTGG TTTACGTTTT
721 GATACGGTAG CGACCTACTC AAAAATTCCG GATTTCCCAA ATCTCACCCA ACAACAGCTG
781 AAGAATTTTG CAGCGGAGTA TACCAAGGGC CCTAATATTC ATCGTTACGT CAATGAAATG
841 AATAAAGAGG TCTTGTCTCA TTACGACATT GCGACTGCCG GTGAAATCTT TGGCGTACCC
901 TTGGATCAAT CGATAAAGTT CTTCGATCGC CGCCGTGATG AGCTGAACAT TGCATTTACC
961 TTTGACTTAA TCAGACTCGA TCGAGACTCT GATCAAAGAT GGCGTCGAAA AGATTGGAAA
1021 TTGTCGCAAT TCCGGCAGAT CATCGATAAC GTTGACCGTA CTGCAGGAGA ATATGGTTGG
1081 AATGCCTTCT TCTTGGATAA CCACGACAAT CCGCGCGCTG TCTCGCACTT TGGCGATGAT
1141 CGCCCACAAT GGCGTGAGCC ATCGGCTAAA GCGCTTGCAA CCTTGACGCT GACTCAACGA
1201 GCAACACCTT TTATTTATCA AGGTTCAGAA TTGGGCATGA CCAATTACCC GTTTAAAGCT
1261 ATTGATGAAT TCGATGATAT TGAGGTGAAA GGTTTTTGGC ATGACTACGT TGAGACAGGA
1321 AAGGTCAAAG CCGACGAGTT CTTGCAAAAT GTACGCCTGA CGAGCAGGGA TAACAGCCGG
1381 ACGCCGTTCC AATGGGATGG GAGCAAAAAT GCAGGATTCA CGAGCGGAAA ACCTTGGTTC
1441 AAGGTCAACC CAAACTACCA GGAAATCAAT GCAGTAAGTC AAGTCACACA ACCCGACTCA
1501 GTATTTAACT ATTATCGTCA GTTGATCAAG ATAAGGCATG ACATCCCGGC ACTGACCTAT
1561 GGTACATACA CCGATTTGGA TCCTGCAAAT GATTCGGTCT ACGCCTATAC ACGCAGCCTT
1621 GGGGCGGAAA AATATCTTGT TGTTGTTAAC TTCAAGGAGC AAATGATGAG ATATAAATTA
1681 CCGGATAATT TATCCATTGA GAAAGTGATT ATAGACAGCA ACAGCAAAAA CGTGGTGAAA
1741 AAGAATGATT CATTACTCGA GCTAAAACCA TGGCAGTCAG GGGTTTATAA ACTAAATCAA
1801 TAA

### SEQ ID NO. 10

BESCHREIBUNG: P. rubrum Isomerase (Variante)
LÄNGE: 1803 Basen oder 600 Aminosäuren

*** SEQUENZ ***

1 ATG CCC CGT CAA GGA TTG AAA ACT GCA CTA GCG ATT TTT CTA ACC ACA
Met Pro Arg Gln Gly Leu Lys Thr Ala Leu Ala Ile Phe Leu Thr Thr

49 TCA TTA TGC ATC TCA TGC CAG CAA GCC TTC GGT ACG CAA CAA CCC TTG
Ser Leu Cys Ile Ser Cys Gln Gln Ala Phe Gly Thr Gln Gln Pro Leu

97 CTT AAC GAA AAG AGT ATC GAA CAG TCG AAA ACC ATA CCT AAA TGG TGG
Leu Asn Glu Lys Ser Ile Glu Gln Ser Lys Thr Ile Pro Lys Trp Trp

145 AAG GAG GCT GTT TTT TAT CAG GTG TAT CCG CGC TCC TTT AAA GAC ACC
Lys Glu Ala Val Phe Tyr Gln Val Tyr Pro Arg Ser Phe Lys Asp Thr

193 AAC GGA GAT GGC ATC GGG GAT ATT AAC GGC ATC ATA GAA AAA TTA GAC
Asn Gly Asp Gly Ile Gly Asp Ile Asn Gly Ile Ile Glu Lys Leu Asp

241 TAT CTA AAA GCC TTG GGG ATT GAT GCC ATT TGG ATC AAC CCA CAT TAT
Tyr Leu Lys Ala Leu Gly Ile Asp Ala Ile Trp Ile Asn Pro His Tyr

289 GAT TCT CCG AAC ACG GAT AAT GGT TAC GAT ATA CGT GAT TAT CGA AAA
Asp Ser Pro Asn Thr Asp Asn Gly Tyr Asp Ile Arg Asp Tyr Arg Lys

337 ATC ATG AAA GAA TAT GGC ACG ATG GAG GAT TTT GAC CGC CTG ATT TCT
Ile Met Lys Glu Tyr Gly Thr Met Glu Asp Phe Asp Arg Leu Ile Ser

385 GAA ATG AAA AAA CGG AAT ATG CGG TTG ATG ATT GAT GTG GTC ATC AAC
Glu Met Lys Lys Arg Asn Met Arg Leu Met Ile Asp Val Val Ile Asn

433 CAC ACC AGC GAT CAA AAC GAA TGG TTT GTT AAA AGT AAA AGC AGT AAG
His Thr Ser Asp Gln Asn Glu Trp Phe Val Lys Ser Lys Ser Ser Lys

481 GAT AAT CCT TAT CGC GGC TAT TAT TTC TGG AAA GAT GCT AAA GAA GGG
Asp Asn Pro Tyr Arg Gly Tyr Tyr Phe Trp Lys Asp Ala Lys Glu Gly

529 CAG GCG CCT AAT AAT TAC CCT TCA TTC TTT GGT GGC TCG GCG TGG CAA
Gln Ala Pro Asn Asn Tyr Pro Ser Phe Phe Gly Gly Ser Ala Trp Gln

577 AAA GAT GAA AAG ACC AAT CAA TAC TAC CTG CAC TAT TTT GCT AAA CAA
Lys Asp Glu Lys Thr Asn Gln Tyr Tyr Leu His Tyr Phe Ala Lys Gln

625 CAG CCT GAC CTA AAC TGG GAT AAT CCC AAA GTC CGT CAA GAT CTT TAT
Gln Pro Asp Leu Asn Trp Asp Asn Pro Lys Val Arg Gln Asp Leu Tyr

673 GCA ATG TTA CGT TTC TGG TTA GAT AAA GGC GTG TCT GGT TTA CGT TTT
Ala Met Leu Arg Phe Trp Leu Asp Lys Gly Val Ser Gly Leu Arg Phe

721 GAT ACG GTA GCG ACC TAC TCA AAA ATT CCG GAT TTC CCA AAT CTC ACC
Asp Thr Val Ala Thr Tyr Ser Lys Ile Pro Asp Phe Pro Asn Leu Thr

769 CAA CAA CAG CTG AAG AAT TTT GCA GCG GAG TAT ACC AAG GGC CCT AAT
Gln Gln Gln Leu Lys Asn Phe Ala Ala Glu Tyr Thr Lys Gly Pro Asn

817 ATT CAT CGT TAC GTC AAT GAA ATG AAT AAA GAG GTC TTG TCT CAT TAC
Ile His Arg Tyr Val Asn Glu Met Asn Lys Glu Val Leu Ser His Tyr

865 GAC ATT GCG ACT GCC GGT GAA ATC TTT GGC GTA CCC TTG GAT CAA TCG
Asp Ile Ala Thr Ala Gly Glu Ile Phe Gly Val Pro Leu Asp Gln Ser

913 ATA AAG TTC TTC GAT CGC CGC CGT GAT GAG CTG AAC ATT GCA TTT ACC
Ile Lys Phe Phe Asp Arg Arg Arg Asp Glu Leu Asn Ile Ala Phe Thr

961 TTT GAC TTA ATC AGA CTC GAT CGA GAC TCT GAT CAA AGA TGG CGT CGA
Phe Asp Leu Ile Arg Leu Asp Arg Asp Ser Asp Gln Arg Trp Arg Arg

1009 AAA GAT TGG AAA TTG TCG CAA TTC CGG CAG ATC ATC GAT AAC GTT GAC
Lys Asp Trp Lys Leu Ser Gln Phe Arg Gln Ile Ile Asp Asn Val Asp

1057 CGT ACT GCA GGA GAA TAT GGT TGG AAT GCC TTC TTC TTG GAT AAC CAC
Arg Thr Ala Gly Glu Tyr Gly Trp Asn Ala Phe Phe Leu Asp Asn His

1105 GAC AAT CCG CGC GCT GTC TCG CAC TTT GGC GAT GAT CGC CCA CAA TGG
Asp Asn Pro Arg Ala Val Ser His Phe Gly Asp Asp Arg Pro Gln Trp

1153 CGT GAG CCA TCG GCT AAA GCG CTT GCA ACC TTG ACG CTG ACT CAA CGA
Arg Glu Pro Ser Ala Lys Ala Leu Ala Thr Leu Thr Leu Thr Gln Arg

1201 GCA ACA CCT TTT ATT TAT CAA GGT TCA GAA TTG GGC ATG ACC AAT TAC
Ala Thr Pro Phe Ile Tyr Gln Gly Ser Glu Leu Gly Met Thr Asn Tyr

1249 CCG TTT AAA GCT ATT GAT GAA TTC GAT GAT ATT GAG GTG AAA GGT TTT
Pro Phe Lys Ala Ile Asp Glu Phe Asp Asp Ile Glu Val Lys Gly Phe

1297 TGG CAT GAC TAC GTT GAG ACA GGA AAG GTC AAA GCC GAC GAG TTC TTG
Trp His Asp Tyr Val Glu Thr Gly Lys Val Lys Ala Asp Glu Phe Leu

1345 CAA AAT GTA CGC CTG ACG AGC AGG GAT AAC AGC CGG ACG CCG TTC CAA
Gln Asn Val Arg Leu Thr Ser Arg Asp Asn Ser Arg Thr Pro Phe Gln

1393 TGG GAT GGG AGC AAA AAT GCA GGA TTC ACG AGC GGA AAA CCT TGG TTC
Trp Asp Gly Ser Lys Asn Ala Gly Phe Thr Ser Gly Lys Pro Trp Phe

1441 AAG GTC AAC CCA AAC TAC CAG GAA ATC AAT GCA GTA AGT CAA GTC ACA
Lys Val Asn Pro Asn Tyr Gln Glu Ile Asn Ala Val Ser Gln Val Thr

1489 CAA CCC GAC TCA GTA TTT AAC TAT TAT CGT CAG TTG ATC AAG ATA AGG
Gln Pro Asp Ser Val Phe Asn Tyr Tyr Arg Gln Leu Ile Lys Ile Arg

1537 CAT GAC ATC CCG GCA CTG ACC TAT GGT ACA TAC ACC GAT TTG GAT CCT
His Asp Ile Pro Ala Leu Thr Tyr Gly Thr Tyr Thr Asp Leu Asp Pro

1585 GCA AAT GAT TCG GTC TAC GCC TAT ACA CGC AGC CTT GGG GCG GAA AAA
Ala Asn Asp Ser Val Tyr Ala Tyr Thr Arg Ser Leu Gly Ala Glu Lys

1633 TAT CTT GTT GTT GTT AAC TTC AAG GAG CAA ATG ATG AGA TAT AAA TTA
Tyr Leu Val Val Val Asn Phe Lys Glu Gln Met Met Arg Tyr Lys Leu

1681 CCG GAT AAT TTA TCC ATT GAG AAA GTG ATT ATA GAC AGC AAC AGC AAA
Pro Asp Asn Leu Ser Ile Glu Lys Val Ile Ile Asp Ser Asn Ser Lys

1729 AAC GTG GTG AAA AAG AAT GAT TCA TTA CTC GAG CTA AAA CCA TGG CAG
Asn Val Val Lys Lys Asn Asp Ser Leu Leu Glu Leu Lys Pro Trp Gln

1777 TCA GGG GTT TAT AAA CTA AAT CAA TAA
Ser Gly Val Tyr Lys Leu Asn Gln ---

### SEQ ID NO. 11

BESCHREIBUNG: SZ 62-Isomerase
LÄNGE: 1794 Basen

*** SEQUENZ ***

1 ATGTCTTTTG TTACGCTACG TACCGGGGTG GCTGTCGCGC TGTCATCTTT GATAATAAGT
61 CTGGCCTGCC CGGCTGTCAG TGCTGCACCA TCCTTGAATC AGGATATTCA CGTTCAAAAG
121 GAAAGTGAAT ATCCTGCATG GTGGAAAGAA GCTGTTTTTT ATCAGATCTA TCCTCGCTCA
181 TTTAAAGACA CCAATGATGA TGGCATTGGC GATATTCGCG GTATTATTGA AAAGCTGGAC
241 TATCTGAAAT CGCTCGGTAT TGACGCTATC TGGATCAATC CCCATTACGA CTCTCCGAAC
301 ACCGATAACG GCTATGACAT CAGTAATTAT CGTCAGATAA TGAAAGAGTA TGGCACAATG
361 GAGGATTTTG ATAGCCTTGT TGCCGAAATG AAAAAACGAA ATATGCGCTT AATGATCGAC
421 GTGGTCATTA ACCATACCAG TGATCAACAC CCGTGGTTTA TTCAGAGTAA AAGCGATAAA
481 AACAACCCTT ATCGTGACTA TTATTTCTGG CGTGACGGAA AAGATAATCA GCCACCTAAT
541 AATTACCCCT CATTTTTCGG CGGCTCGGCA TGGCAAAAAG ATGCAAAGTC AGGACAGTAC
601 TATTTACACT ATTTTGCCAG ACAGCAACCT GATCTCAACT GGGATAACCC GAAAGTACGT
661 GAGGATCTTT ACGCAATGCT CCGCTTCTGG CTGGATAAAG GCGTTTCAGG CATGCGATTT
721 GATACGGTGG CAACTTATTC CAAAATCCCG GGATTTCCCA ATCTGACACC TGAACAACAG
781 AAAAATTTTG CTGAACAATA CACCATGGGD CCTAATATTC ATCGATACAT TCAGGAAATG
841 AACCGGAAAG TTCTGTCCCG GTATGATGTG GCCACCGCGG GTGAAATTTT TGGCGTCCCG
901 CTGGATCGTT CGTCGCAGTT TTTTGATCGC CGCCGACATG AGCTGAATAT GGCGTTTATG
961 TTTGACCTCA TTCGTCTCGA TCGCGACAGC AATGAACGCT GGCGTCACAA GTCGTGGTCG
1021 CTCTCTCAGT TCCGCCAGAT CATCAGCAAA ATGGATGTCA CGGTCGGAAA GTATGGCTGG
1081 AACACGTTCT TCTTAGACAA CCATGACAAC CCCCGTGCGG TATCTCACTT CGGGGATGAC
1141 AGGCCGCAAT GGCGGGAGGC GTCGGCTAAG GCACTGGCGA CGATTACCCT CACTCAGCGG
1201 GCGACGCCGT TTATTTATCA GGGTTCAGAG CTGGGAATGA CTAATTATCC CTTCAGGCAA
1261 CTCAACGAAT TTGACGACAT CGAGGTCAAA GGTTTCTGGC AGGATTATGT CCAGAGTGGA
1321 AAAGTCACGG CCACAGAGTT TCTCGATAAT GTGCGCCTGA CGAGCCGCGA TAACAGCAGA
1381 ACACCTTTCC AGTGGAATGA CACCCTGAAT GCTGGTTTTA CTCGCGGAAA GCCGTGGTTT
1441 CACATCAACC CAAACTATGT GGAGATCAAC SCCGAACGCG AAGAAACCCG CGAAGATTCA
1501 GTGCTGAATT ACTATAAAAA AATGATTCAG CTACGCCACC ATATCCCTGC TCTGGTATAT
1561 GGCGCCTATC AGGATCTTAA TCCACAGGAC AATACCGTTT ATGCCTATAC CCGAACGCTG
1621 GGTAACGAGC GTTATCTGGT CGTGGTGAAC TTTAAGGAGT ACCCGGTCCG CTATACTCTC
1681 CCGGCTAATG ATGCCATCGA GGAAGTGGTC ATTGATACTC AGCAGCAAGG TGCGCCGCAC
1741 AGCACATCCC TGTCATTGAG CCCCTGGCAG GCAGGTGCGT ATAAGCTGCG GTAA

### SEQ ID NO. 12

BESCHREIBUNG: SZ 62-Isomerase
LÄNGE: 1794 Basen oder 597 Aminosäuren

*** SEQUENZ ***

1 ATG TCT TTT GTT ACG CTA CGT ACC GGG GTG GCT GTC GCG CTG TCA TCT
Met Ser Phe Val Thr Leu Arg Thr Gly Val Ala Val Ala Leu Ser Ser

49 TTG ATA ATA AGT CTG GCC TGC CCG GCT GTC AGT GCT GCA CCA TCC TTG
Leu Ile Ile Ser Leu Ala Cys Pro Ala Val Ser Ala Ala Pro Ser Leu

97 AAT CAG GAT ATT CAC GTT CAA AAG GAA AGT GAA TAT CCT GCA TGG TGG
Asn Gln Asp Ile His Val Gln Lys Glu Ser Glu Tyr Pro Ala Trp Trp

145 AAA GAA GCT GTT TTT TAT CAG ATC TAT CCT CGC TCA TTT AAA GAC ACC
Lys Glu Ala Val Phe Tyr Gln Ile Tyr Pro Arg Ser Phe Lys Asp Thr

193 AAT GAT GAT GGC ATT GGC GAT ATT CGC GGT ATT ATT GAA AAG CTG GAC
Asn Asp Asp Gly Ile Gly Asp Ile Arg Gly Ile Ile Glu Lys Leu Asp

241 TAT CTG AAA TCG CTC GGT ATT GAC GCT ATC TGG ATC AAT CCC CAT TAC
Tyr Leu Lys Ser Leu Gly Ile Asp Ala Ile Trp Ile Asn Pro His Tyr

289 GAC TCT CCG AAC ACC GAT AAC GGC TAT GAC ATC AGT AAT TAT CGT CAG
Asp Ser Pro Asn Thr Asp Asn Gly Tyr Asp Ile Ser Asn Tyr Arg Gln

337 ATA ATG AAA GAG TAT GGC ACA ATG GAG GAT TTT GAT AGC CTT GTT GCC
Ile Met Lys Glu Tyr Gly Thr Met Glu Asp Phe Asp Ser Leu Val Ala

385 GAA ATG AAA AAA CGA AAT ATG CGC TTA ATG ATC GAC GTG GTC ATT AAC
Glu Met Lys Lys Arg Asn Met Arg Leu Met Ile Asp Val Val Ile Asn

433 CAT ACC AGT GAT CAA CAC CCG TGG TTT ATT CAG AGT AAA AGC GAT AAA
His Thr Ser Asp Gln His Pro Trp Phe Ile Gln Ser Lys Ser Asp Lys

481 AAC AAC CCT TAT CGT GAC TAT TAT TTC TGG CGT GAC GGA AAA GAT AAT
Asn Asn Pro Tyr Arg Asp Tyr Tyr Phe Trp Arg Asp Gly Lys Asp Asn

529 CAG CCA CCT AAT AAT TAC CCC TCA TTT TTC GGC GGC TCG GCA TGG CAA
Gln Pro Pro Asn Asn Tyr Pro Ser Phe Phe Gly Gly Ser Ala Trp Gln

577 AAA GAT GCA AAG TCA GGA CAG TAC TAT TTA CAC TAT TTT GCC AGA CAG
Lys Asp Ala Lys Ser Gly Gln Tyr Tyr Leu His Tyr Phe Ala Arg Gln

625 CAA CCT GAT CTC AAC TGG GAT AAC CCG AAA GTA CGT GAG GAT CTT TAC
Gln Pro Asp Leu Asn Trp Asp Asn Pro Lys Val Arg Glu Asp Leu Tyr

673 GCA ATG CTC CGC TTC TGG CTG GAT AAA GGC GTT TCA GGC ATG CGA TTT
Ala Met Leu Arg Phe Trp Leu Asp Lys Gly Val Ser Gly Met Arg Phe

721 GAT ACG GTG GCA ACT TAT TCC AAA ATC CCG GGA TTT CCC AAT CTG ACA
Asp Thr Val Ala Thr Tyr Ser Lys Ile Pro Gly Phe Pro Asn Leu Thr

769 CCT GAA CAA CAG AAA AAT TTT GCT GAA CAA TAC ACC ATG GGD CCT AAT
Pro Glu Gln Gln Lys Asn Phe Ala Glu Gln Tyr Thr Met ? Pro Asn

817 ATT CAT CGA TAC ATT CAG GAA ATG AAC CGG AAA GTT CTG TCC CGG TAT
Ile His Arg Tyr Ile Gln Glu Met Asn Arg Lys Val Leu Ser Arg Tyr

865 GAT GTG GCC ACC GCG GGT GAA ATT TTT GGC GTC CCG CTG GAT CGT TCG
Asp Val Ala Thr Ala Gly Glu Ile Phe Gly Val Pro Leu Asp Arg Ser

913 TCG CAG TTT TTT GAT CGC CGC CGA CAT GAG CTG AAT ATG GCG TTT ATG
Ser Gln Phe Phe Asp Arg Arg Arg His Glu Leu Asn Met Ala Phe Met

961 TTT GAC CTC ATT CGT CTC GAT CGC GAC AGC AAT GAA CGC TGG CGT CAC
Phe Asp Leu Ile Arg Leu Asp Arg Asp Ser Asn Glu Arg Trp Arg His

1009 AAG TCG TGG TCG CTC TCT CAG TTC CGC CAG ATC ATC AGC AAA ATG GAT
Lys Ser Trp Ser Leu Ser Gln Phe Arg Gln Ile Ile Ser Lys Met Asp

1057 GTC ACG GTC GGA AAG TAT GGC TGG AAC ACG TTC TTC TTA GAC AAC CAT
Val Thr Val Gly Lys Tyr Gly Trp Asn Thr Phe Phe Leu Asp Asn His

1105 GAC AAC CCC CGT GCG GTA TCT CAC TTC GGG GAT GAC AGG CCG CAA TGG
Asp Asn Pro Arg Ala Val Ser His Phe Gly Asp Asp Arg Pro Gln Trp

1153 CGG GAG GCG TCG GCT AAG GCA CTG GCG ACG ATT ACC CTC ACT CAG CGG
Arg Glu Ala Ser Ala Lys Ala Leu Ala Thr Ile Thr Leu Thr Gln Arg

1201 GCG ACG CCG TTT ATT TAT CAG GGT TCA GAG CTG GGA ATG ACT AAT TAT
Ala Thr Pro Phe Ile Tyr Gln Gly Ser Glu Leu Gly Met Thr Asn Tyr

1249 CCC TTC AGG CAA CTC AAC GAA TTT GAC GAC ATC GAG GTC AAA GGT TTC
Pro Phe Arg Gln Leu Asn Glu Phe Asp Asp Ile Glu Val Lys Gly Phe

1297 TGG CAG GAT TAT GTC CAG AGT GGA AAA GTC ACG GCC ACA GAG TTT CTC
Trp Gln Asp Tyr Val Gln Ser Gly Lys Val Thr Ala Thr Glu Phe Leu

1345 GAT AAT GTG CGC CTG ACG AGC CGC GAT AAC AGC AGA ACA CCT TTC CAG
Asp Asn Val Arg Leu Thr Ser Arg Asp Asn Ser Arg Thr Pro Phe Gln

1393 TGG AAT GAC ACC CTG AAT GCT GGT TTT ACT CGC GGA AAG CCG TGG TTT
Trp Asn Asp Thr Leu Asn Ala Gly Phe Thr Arg Gly Lys Pro Trp Phe

1441 CAC ATC AAC CCA AAC TAT GTG GAG ATC AAC SCC GAA CGC GAA GAA ACC
His Ile Asn Pro Asn Tyr Val Glu Ile Asn ? Glu Arg Glu Glu Thr

1489 CGC GAA GAT TCA GTG CTG AAT TAC TAT AAA AAA ATG ATT CAG CTA CGC
Arg Glu Asp Ser Val Leu Asn Tyr Tyr Lys Lys Met Ile Gln Leu Arg

1537 CAC CAT ATC CCT GCT CTG GTA TAT GGC GCC TAT CAG GAT CTT AAT CCA
His His Ile Pro Ala Leu Val Tyr Gly Ala Tyr Gln Asp Leu Asn Pro

1585 CAG GAC AAT ACC GTT TAT GCC TAT ACC CGA ACG CTG GGT AAC GAG CGT
Gln Asp Asn Thr Val Tyr Ala Tyr Thr Arg Thr Leu Gly Asn Glu Arg

1633 TAT CTG GTC GTG GTG AAC TTT AAG GAG TAC CCG GTC CGC TAT ACT CTC
Tyr Leu Val Val Val Asn Phe Lys Glu Tyr Pro Val Arg Tyr Thr Leu

1681 CCG GCT AAT GAT GCC ATC GAG GAA GTG GTC ATT GAT ACT CAG CAG CAA
Pro Ala Asn Asp Ala Ile Glu Glu Val Val Ile Asp Thr Gln Gln Gln

1729 GGT GCG CCG CAC AGC ACA TCC CTG TCA TTG AGC CCC TGG CAG GCA GGT
Gly Ala Pro His Ser Thr Ser Leu Ser Leu Ser Pro Trp Gln Ala Gly

1777 GCG TAT AAG CTG CGG TAA
Ala Tyr Lys Leu Arg ---

### SEQ ID NO. 13

BESCHREIBUNG: MX 45-Isomerase
LÄNGE: 1782 Basen

*** S E Q U E N Z ***

1 ATGCTTATGA AGAGATTATT CGCCGCGTCT CTGATGCTTG CTTTTTCAAG CGTCTCCTCT
61 GTGAGGGCTG AGGAGGCCGT AAAGCCGGGC GCGCCATGGT GGAAAAGTGC TGTCTTCTAT
121 CAGGTCTATC CGCGCTCGTT CAAGGATACC AACGGTGATG GGATCGGCGA TTTCAAAGGA
181 CTGACGGAGA AGCTCGACTA TCTCAAGGGG CTCGGCATAG ACGCCATCTG GATCAATCCA
241 CATTACGCGT CTCCCAACAC CGATAATGGC TACGATATCA GCGACTATCG AGAGGTCATG
301 AAGGAATATG GGACGATGGA GGACTTCGAT CGTCTGATGG CTGAGTTGAA GAAGCGCGGC
361 ATGCGGCTCA TGGTTGATGT CGTGATCAAC CATTCGAGTG ACCAACACGA ATGGTTCAAG
421 AGCAGCCGGG CCTCCAAAGA CAATCCCTAC CGTGACTATT ATTTCTGGCG TGACGGCAAA
481 GACGGTCACG AGCCAAACAA TTACCCTTCC TTCTTCGGCG GTTCGGCATG GGAGAAGGAC
541 CCCGTAACCG GGCAATATTA CCTGCATTAT TTCGGTCGTC AGCAGCCAGA TCTGAACTGG
601 GACACGCCGA AGCTTCGCGA GGAACTCTAT GCGATGCTGC GGTTCTGGCT CGACAAGGGC
661 GTATCAGGCA TGCGGTTCGA TACGGTGGCT ACCTACTCGA AGACACCGGG TTTCCCGGAT
721 CTGACACCGG AGCAGATGAA GAACTTCGCG GAGGCCTATA CCCAGGGGCC GAACCTTCAT
781 CGTTACCTGC AGGAAATGCA CGAGAAGGTC TTCGATCATT ATGACGCGGT CACGGCCGGC
841 GAAATCTTCG GCGCTCCGCT CAATCAAGTG CCGCTGTTCA TCGACAGCCG GAGGAAAGAG
901 CTGGATATGG CTTTCACCTT CGATCTGATC CGTTATGATC GCGCACTGGA TCGTTGGCAT
961 ACCATTCCGC GTACCTTAGC GGACTTCCGT CAAACGATCG ATAAGGTCGA CGCCATCGCG
1021 GGCGAATATG GCTGGAACAC GTTCTTCCTC GGCAATCACG ACAATCCCCG TGCGGTATCG
1081 CATTTTGGTG ACGATCGGCC GCAATGGCGC GAAGCCTCGG CCAAGGCTCT GGCCACCGTC
1141 ACCTTGACCC AGCGAGGAAC GCCGTTCATC TTCCAAGGAG ATGAACTCGG AATGACCAAC
1201 TACCCCTTCA AGACGCTGCA GGACTTTGAT GATATCNNNN NNNNNNNNNN NNNNNNNNNN
1261 NNNNNNNNNN NNNNNNNNNN NNNNNNNNNN NNNNNNNNNN NNNNNNNNNN NNNNNNNNNN
1321 NNNNNNNNNN NTGTGGCGTT GACTAGCCGA GCAAACGCCC GCACGCCCTT TCAATGGGAT
1381 GACAGTGCTA ATGCGGGATT CACAACTGGC AAGCCTTGGC TAAAGGTCAA TCCAAACTAC
1441 ACTGAGATCA ACGCCGCGCG GGAAATTGGC GATCCTAAAT CGGTCTACAG CTTTTACCGC
1501 AACCTGATCT CAATCCGGCA TGAAACTCCC GCTCTTTCGA CCGGGAGCTA TCGCGACATC
1561 GATCCGAGTA ATGCCGATGT CTATGCCTAT ACGCGCAGCC AGGATGGCGA GACCTATCTG
1621 GTCGTAGTCA ACTTCAAGGC AGAGCCAAGG AGTTTCACGC TTCCGGACGG CATGCATATT
1681 GCCGAAACCC TGATTGAGAG CAGTTCGCCA GCAGCTCCGG CGGCGGGGGC TGCAAGCCTT
1741 GAGCTGCAGC CTTGGCAGTC CGGCATCTAC AAGGTGAAGT AA

### SEQ ID NO. 14

BESCHREIBUNG: MX 45-Isomerase
LÄNGE: 1782 Basen oder 593 Aminosäuren

*** SEQUENZ ***

1 ATG CTT ATG AAG AGA TTA TTC GCC GCG TCT CTG ATG CTT GCT TTT TCA
Met Leu Met Lys Arg Leu Phe Ala Ala Ser Leu Met Leu Ala Phe Ser

49 AGC GTC TCC TCT GTG AGG GCT GAG GAG GCC GTA AAG CCG GGC GCG CCA
Ser Val Ser Ser Val Arg Ala Glu Glu Ala Val Lys Pro Gly Ala Pro

97 TGG TGG AAA AGT GCT GTC TTC TAT CAG GTC TAT CCG CGC TCG TTC AAG
Trp Trp Lys Ser Ala Val Phe Tyr Gln Val Tyr Pro Arg Ser Phe Lys

145 GAT ACC AAC GGT GAT GGG ATC GGC GAT TTC AAA GGA CTG ACG GAG AAG
Asp Thr Asn Gly Asp Gly Ile Gly Asp Phe Lys Gly Leu Thr Glu Lys

193 CTC GAC TAT CTC AAG GGG CTC GGC ATA GAC GCC ATC TGG ATC AAT CCA
Leu Asp Tyr Leu Lys Gly Leu Gly Ile Asp Ala Ile Trp Ile Asn Pro

241 CAT TAC GCG TCT CCC AAC ACC GAT AAT GGC TAC GAT ATC AGC GAC TAT
His Tyr Ala Ser Pro Asn Thr Asp Asn Gly Tyr Asp Ile Ser Asp Tyr

289 CGA GAG GTC ATG AAG GAA TAT GGG ACG ATG GAG GAC TTC GAT CGT CTG
Arg Glu Val Met Lys Glu Tyr Gly Thr Met Glu Asp Phe Asp Arg Leu

337 ATG GCT GAG TTG AAG AAG CGC GGC ATG CGG CTC ATG GTT GAT GTC GTG
Met Ala Glu Leu Lys Lys Arg Gly Met Arg Leu Met Val Asp Val Val

385 ATC AAC CAT TCG AGT GAC CAA CAC GAA TGG TTC AAG AGC AGC CGG GCC
Ile Asn His Ser Ser Asp Gln His Glu Trp Phe Lys Ser Ser Arg Ala

433 TCC AAA GAC AAT CCC TAC CGT GAC TAT TAT TTC TGG CGT GAC GGC AAA
Ser Lys Asp Asn Pro Tyr Arg Asp Tyr Tyr Phe Trp Arg Asp Gly Lys

481 GAC GGT CAC GAG CCA AAC AAT TAC CCT TCC TTC TTC GGC GGT TCG GCA
Asp Gly His Glu Pro Asn Asn Tyr Pro Ser Phe Phe Gly Gly Ser Ala

529 TGG GAG AAG GAC CCC GTA ACC GGG CAA TAT TAC CTG CAT TAT TTC GGT
Trp Glu Lys Asp Pro Val Thr Gly Gln Tyr Tyr Leu His Tyr Phe Gly

577 CGT CAG CAG CCA GAT CTG AAC TGG GAC ACG CCG AAG CTT CGC GAG GAA
Arg Gln Gln Pro Asp Leu Asn Trp Asp Thr Pro Lys Leu Arg Glu Glu

625 CTC TAT GCG ATG CTG CGG TTC TGG CTC GAC AAG GGC GTA TCA GGC ATG
Leu Tyr Ala Met Leu Arg Phe Trp Leu Asp Lys Gly Val Ser Gly Met

673 CGG TTC GAT ACG GTG GCT ACC TAC TCG AAG ACA CCG GGT TTC CCG GAT
Arg Phe Asp Thr Val Ala Thr Tyr Ser Lys Thr Pro Gly Phe Pro Asp

721 CTG ACA CCG GAG CAG ATG AAG AAC TTC GCG GAG GCC TAT ACC CAG GGG
Leu Thr Pro Glu Gln Met Lys Asn Phe Ala Glu Ala Tyr Thr Gln Gly

769 CCG AAC CTT CAT CGT TAC CTG CAG GAA ATG CAC GAG AAG GTC TTC GAT
Pro Asn Leu His Arg Tyr Leu Gln Glu Met His Glu Lys Val Phe Asp

817 CAT TAT GAC GCG GTC ACG GCC GGC GAA ATC TTC GGC GCT CCG CTC AAT
His Tyr Asp Ala Val Thr Ala Gly Glu Ile Phe Gly Ala Pro Leu Asn

865 CAA GTG CCG CTG TTC ATC GAC AGC CGG AGG AAA GAG CTG GAT ATG GCT
Gln Val Pro Leu Phe Ile Asp Ser Arg Arg Lys Glu Leu Asp Met Ala

913 TTC ACC TTC GAT CTG ATC CGT TAT GAT CGC GCA CTG GAT CGT TGG CAT
Phe Thr Phe Asp Leu Ile Arg Tyr Asp Arg Ala Leu Asp Arg Trp His

961 ACC ATT CCG CGT ACC TTA GCG GAC TTC CGT CAA ACG ATC GAT AAG GTC
Thr Ile Pro Arg Thr Leu Ala Asp Phe Arg Gln Thr Ile Asp Lys Val

1009 GAC GCC ATC GCG GGC GAA TAT GGC TGG AAC ACG TTC TTC CTC GGC AAT
Asp Ala Ile Ala Gly Glu Tyr Gly Trp Asn Thr Phe Phe Leu Gly Asn

1057 CAC GAC AAT CCC CGT GCG GTA TCG CAT TTT GGT GAC GAT CGG CCG CAA
His Asp Asn Pro Arg Ala Val Ser His Phe Gly Asp Asp Arg Pro Gln

1105 TGG CGC GAA GCC TCG GCC AAG GCT CTG GCC ACC GTC ACC TTG ACC CAG
Trp Arg Glu Ala Ser Ala Lys Ala Leu Ala Thr Val Thr Leu Thr Gln

1153 CGA GGA ACG CCG TTC ATC TTC CAA GGA GAT GAA CTC GGA ATG ACC AAC
Arg Gly Thr Pro Phe Ile Phe Gln Gly Asp Glu Leu Gly Met Thr Asn

1201 TAC CCC TTC AAG ACG CTG CAG GAC TTT GAT GAT ATC NNN NNN NNN NNN
Tyr Pro Phe Lys Thr Leu Gln Asp Phe Asp Asp Ile ? ? ? ?

1249 NNN NNN NNN NNN NNN NNN NNN NNN NNN NNN NNN NNN NNN NNN NNN NNN
? ? ? ? ? ? ? ? ? ? ? ? ? ? ? ?

1297 NNN NNN NNN NNN NNN NNN NNN NNN NNN NNN NNN NNT GTG GCG TTG ACT
? ? ? ? ? ? ? ? ? ? ? ? Val Ala Leu Thr

1345 AGC CGA GCA AAC GCC CGC ACG CCC TTT CAA TGG GAT GAC AGT GCT AAT
Ser Arg Ala Asn Ala Arg Thr Pro Phe Gln Trp Asp Asp Ser Ala Asn

1393 GCG GGA TTC ACA ACT GGC AAG CCT TGG CTA AAG GTC AAT CCA AAC TAC
Ala Gly Phe Thr Thr Gly Lys Pro Trp Leu Lys Val Asn Pro Asn Tyr

1441 ACT GAG ATC AAC GCC GCG CGG GAA ATT GGC GAT CCT AAA TCG GTC TAC
Thr Glu Ile Asn Ala Ala Arg Glu Ile Gly Asp Pro Lys Ser Val Tyr

1489 AGC TTT TAC CGC AAC CTG ATC TCA ATC CGG CAT GAA ACT CCC GCT CTT
Ser Phe Tyr Arg Asn Leu Ile Ser Ile Arg His Glu Thr Pro Ala Leu

1537 TCG ACC GGG AGC TAT CGC GAC ATC GAT CCG AGT AAT GCC GAT GTC TAT
Ser Thr Gly Ser Tyr Arg Asp Ile Asp Pro Ser Asn Ala Asp Val Tyr

1585 GCC TAT ACG CGC AGC CAG GAT GGC GAG ACC TAT CTG GTC GTA GTC AAC
Ala Tyr Thr Arg Ser Gln Asp Gly Glu Thr Tyr Leu Val Val Val Asn

1633 TTC AAG GCA GAG CCA AGG AGT TTC ACG CTT CCG GAC GGC ATG CAT ATT
Phe Lys Ala Glu Pro Arg Ser Phe Thr Leu Pro Asp Gly Met His Ile

1681 GCC GAA ACC CTG ATT GAG AGC AGT TCG CCA GCA GCT CCG GCG GCG GGG
Ala Glu Thr Leu Ile Glu Ser Ser Ser Pro Ala Ala Pro Ala Ala Gly

1729 GCT GCA AGC CTT GAG CTG CAG CCT TGG CAG TCC GGC ATC TAC AAG GTG
Ala Ala Ser Leu Glu Leu Gln Pro Trp Gln Ser Gly Ile Tyr Lys Val

1777 AAG TAA
Lys ---

### SEQ ID NO. 15

BESCHREIBUNG: MX 45-Palatinose-Hydrolase
LÄNGE: 1704 Basen

*** SEQUENZ ***

1 ATGACTGAAA AGTTATCCTT CGAGTCGACA ACAATCTCGC GTCGCTGGTG GAAAGAGGCT
61 GTTGTCTATC AGGTGTATCC CCGCTCGTTC CAGGATTCGA ACGGGGACGG CATCGGCGAC
121 CTTCCGGGCA TAACTGCGAG GCTAGATTAC ATCCTCGGTC TAGGCGTTAG TGTCATCTGG
181 CTCAGCCCCC ATTTCGACTC TCCGAATGCT GACAACGGCT ACGATATCCG TGACTATCGC
241 AAGGTGATGC GCGAATTCGG CACCATGGCG GATTTCGATC ACCTGCTGGC CGAGACGAAA
301 AAGCGCGGCA TGCGGCTGAT CATCGATCTC GTCGTCAACC ATACCAGCGA CGAGCATGTC
361 TGGTTTGCCG AAAGCCGGGC CTCGAAAAAC AGCCCGTACC GTGATTACTA CATCTGGCAT
421 CCCGGCCGGG ACGGCGCCGA GCCGAACGAC TGGCGCTCAT TTTTCTCGGG CTCGGCATGG
481 ACTTTCGACC AGCCAACCGG CGAATACTAC ATGCATCTTT TCGCCGATAA ACAGCCGGAT
541 ATCAACTGGG ACAATCCGGC TGTGCGCGCC GATGTCTATG ACATCATGCG CTTTTGGCTG
601 GACAAGGGCG TCGACGGATT CCGCATGGAT GTCATCCCCT TCATCTCCAA GCAAGACGGC
661 CTGCCCGACT ATCCTGACCA TCATCGCGGC GCGCCGCAGT TTTTCCACGG TTCGGGTCCC
721 CGCTTGCACG ACTATCTTCA GGAAATGAAC CGCGAGGTAT TGTCGCATTA CGATGTGATG
781 ACGGTTGGCG AGGCCTTCGG TGTGACGGCG GATGCGACGC CGCTTCTGGT CGACGAACGG
841 CGCCGCGAAC TGAACATGAT CTTCAATTTC GACGCCGTGC GCATCGGCCG TGGCGAGACC
901 TGGCACACTA AGCCTTGGGC CCTGCCGGAA CTTAAGGCGA TCTATGCCCG TCTGGACGCT
961 GCGACCGACC AGCACTGCTG GGGTACGGTC TTTCTCTCCA ACCACGACAA TCCTCGTCTC
1021 GTCTCCCGGT TCGGTGATGA TCATCCTGAC TGGCGGGTGG CGTCGGCCAA GGTTCTTGCC
1081 ACACTTCTCC TAACGCTGAA GGGCACGCCT TTCATCTACC AAGGCGATGA ATTGGGCATG
1141 ACCAACTATC CTCGGCTCGG TCGAGGAGAC GACGATATCG AGGTGCGCAA CGCCTGGCAG
1201 GCTGAGGTCA TGACCGGTAA GGCGGATGCA GCCGAATTTC TCGGGGAGAT GCTGAAGATT
1261 TCCCGCGATC ATTCCCGCAC ACCGATGCAA TGGGACGCCA GTCTCGACGG TGGTTTCACT
1321 CGGGGTGAAA AGCCCTGGCT ATCGGTCAAT CCGAACTATC GGGCGATCAA TGCGGATGCG
1381 GCACTCGCCG ATCCCGATTC GATCTACCAT TATTACGCCG CACTCATCCG TTTCCGGCGC
1441 GAGACACCGG CGCTCATCTA CGGCGATTAT GACGACTTGG CGCCGGATCA TCCGCACCTC
1501 TTCGTCTATA CAAGAACATT GGGGTCCGAG CGCTATCTGG TCGCGCTTAA CTTCTCCGGC
1561 GATGCGCAGG CACTTGTTCT CCCGACAGAC CTGAGCGCCG CGTCACCTGT TATCGGGCGC
1621 GCCCCGCAAG TGGACCGCAT GCAGCATGAT GCTGCACGGA TCGAGCTGAT GGGTTGGGAA
1681 GCGCGGGTCT ACCACTGCGC ATGA

### SEQ ID NO. 16

BESCHREIBUNG: MX 45-Palatinose-Hydrolase
LÄNGE: 1704 Basen oder 567 Aminosäuren

*** SEQUENZ ***

1 ATG ACT GAA AAG TTA TCC TTC GAG TCG ACA ACA ATC TCG CGT CGC TGG
Met Thr Glu Lys Leu Ser Phe Glu Ser Thr Thr Ile Ser Arg Arg Trp

49 TGG AAA GAG GCT GTT GTC TAT CAG GTG TAT CCC CGC TCG TTC CAG GAT
Trp Lys Glu Ala Val Val Tyr Gln Val Tyr Pro Arg Ser Phe Gln Asp

97 TCG AAC GGG GAC GGC ATC GGC GAC CTT CCG GGC ATA ACT GCG AGG CTA
Ser Asn Gly Asp Gly Ile Gly Asp Leu Pro Gly Ile Thr Ala Arg Leu

145 GAT TAC ATC CTC GGT CTA GGC GTT AGT GTC ATC TGG CTC AGC CCC CAT
Asp Tyr Ile Leu Gly Leu Gly Val Ser Val Ile Trp Leu Ser Pro His

193 TTC GAC TCT CCG AAT GCT GAC AAC GGC TAC GAT ATC CGT GAC TAT CGC
Phe Asp Ser Pro Asn Ala Asp Asn Gly Tyr Asp Ile Arg Asp Tyr Arg

241 AAG GTG ATG CGC GAA TTC GGC ACC ATG GCG GAT TTC GAT CAC CTG CTG
Lys Val Met Arg Glu Phe Gly Thr Met Ala Asp Phe Asp His Leu Leu

289 GCC GAG ACG AAA AAG CGC GGC ATG CGG CTG ATC ATC GAT CTC GTC GTC
Ala Glu Thr Lys Lys Arg Gly Met Arg Leu Ile Ile Asp Leu Val Val

337 AAC CAT ACC AGC GAC GAG CAT GTC TGG TTT GCC GAA AGC CGG GCC TCG
Asn His Thr Ser Asp Glu His Val Trp Phe Ala Glu Ser Arg Ala Ser

385 AAA AAC AGC CCG TAC CGT GAT TAC TAC ATC TGG CAT CCC GGC CGG GAC
Lys Asn Ser Pro Tyr Arg Asp Tyr Tyr Ile Trp His Pro Gly Arg Asp

433 GGC GCC GAG CCG AAC GAC TGG CGC TCA TTT TTC TCG GGC TCG GCA TGG
Gly Ala Glu Pro Asn Asp Trp Arg Ser Phe Phe Ser Gly Ser Ala Trp

481 ACT TTC GAC CAG CCA ACC GGC GAA TAC TAC ATG CAT CTT TTC GCC GAT
Thr Phe Asp Gln Pro Thr Gly Glu Tyr Tyr Met His Leu Phe Ala Asp

529 AAA CAG CCG GAT ATC AAC TGG GAC AAT CCG GCT GTG CGC GCC GAT GTC
Lys Gln Pro Asp Ile Asn Trp Asp Asn Pro Ala Val Arg Ala Asp Val

577 TAT GAC ATC ATG CGC TTT TGG CTG GAC AAG GGC GTC GAC GGA TTC CGC
Tyr Asp Ile Met Arg Phe Trp Leu Asp Lys Gly Val Asp Gly Phe Arg

625 ATG GAT GTC ATC CCC TTC ATC TCC AAG CAA GAC GGC CTG CCC GAC TAT
Met Asp Val Ile Pro Phe Ile Ser Lys Gln Asp Gly Leu Pro Asp Tyr

673 CCT GAC CAT CAT CGC GGC GCG CCG CAG TTT TTC CAC GGT TCG GGT CCC
Pro Asp His His Arg Gly Ala Pro Gln Phe Phe His Gly Ser Gly Pro

721 CGC TTG CAC GAC TAT CTT CAG GAA ATG AAC CGC GAG GTA TTG TCG CAT
Arg Leu His Asp Tyr Leu Gln Glu Met Asn Arg Glu Val Leu Ser His

769 TAC GAT GTG ATG ACG GTT GGC GAG GCC TTC GGT GTG ACG GCG GAT GCG
Tyr Asp Val Met Thr Val Gly Glu Ala Phe Gly Val Thr Ala Asp Ala

817 ACG CCG CTT CTG GTC GAC GAA CGG CGC CGC GAA CTG AAC ATG ATC TTC
Thr Pro Leu Leu Val Asp Glu Arg Arg Arg Glu Leu Asn Met Ile Phe

865 AAT TTC GAC GCC GTG CGC ATC GGC CGT GGC GAG ACC TGG CAC ACT AAG
Asn Phe Asp Ala Val Arg Ile Gly Arg Gly Glu Thr Trp His Thr Lys

913 CCT TGG GCC CTG CCG GAA CTT AAG GCG ATC TAT GCC CGT CTG GAC GCT
Pro Trp Ala Leu Pro Glu Leu Lys Ala Ile Tyr Ala Arg Leu Asp Ala

961 GCG ACC GAC CAG CAC TGC TGG GGT ACG GTC TTT CTC TCC AAC CAC GAC
Ala Thr Asp Gln His Cys Trp Gly Thr Val Phe Leu Ser Asn His Asp

1009 AAT CCT CGT CTC GTC TCC CGG TTC GGT GAT GAT CAT CCT GAC TGG CGG
Asn Pro Arg Leu Val Ser Arg Phe Gly Asp Asp His Pro Asp Trp Arg

1057 GTG GCG TCG GCC AAG GTT CTT GCC ACA CTT CTC CTA ACG CTG AAG GGC
Val Ala Ser Ala Lys Val Leu Ala Thr Leu Leu Leu Thr Leu Lys Gly

1105 ACG CCT TTC ATC TAC CAA GGC GAT GAA TTG GGC ATG ACC AAC TAT CCT
Thr Pro Phe Ile Tyr Gln Gly Asp Glu Leu Gly Met Thr Asn Tyr Pro

1153 CGG CTC GGT CGA GGA GAC GAC GAT ATC GAG GTG CGC AAC GCC TGG CAG
Arg Leu Gly Arg Gly Asp Asp Asp Ile Glu Val Arg Asn Ala Trp Gln

1201 GCT GAG GTC ATG ACC GGT AAG GCG GAT GCA GCC GAA TTT CTC GGG GAG
Ala Glu Val Met Thr Gly Lys Ala Asp Ala Ala Glu Phe Leu Gly Glu

1249 ATG CTG AAG ATT TCC CGC GAT CAT TCC CGC ACA CCG ATG CAA TGG GAC
Met Leu Lys Ile Ser Arg Asp His Ser Arg Thr Pro Met Gln Trp Asp

1297 GCC AGT CTC GAC GGT GGT TTC ACT CGG GGT GAA AAG CCC TGG CTA TCG
Ala Ser Leu Asp Gly Gly Phe Thr Arg Gly Glu Lys Pro Trp Leu Ser

1345 GTC AAT CCG AAC TAT CGG GCG ATC AAT GCG GAT GCG GCA CTC GCC GAT
Val Asn Pro Asn Tyr Arg Ala Ile Asn Ala Asp Ala Ala Leu Ala Asp

1393 CCC GAT TCG ATC TAC CAT TAT TAC GCC GCA CTC ATC CGT TTC CGG CGC
Pro Asp Ser Ile Tyr His Tyr Tyr Ala Ala Leu Ile Arg Phe Arg Arg

1441 GAG ACA CCG GCG CTC ATC TAC GGC GAT TAT GAC GAC TTG GCG CCG GAT
Glu Thr Pro Ala Leu Ile Tyr Gly Asp Tyr Asp Asp Leu Ala Pro Asp

1489 CAT CCG CAC CTC TTC GTC TAT ACA AGA ACA TTG GGG TCC GAG CGC TAT
His Pro His Leu Phe Val Tyr Thr Arg Thr Leu Gly Ser Glu Arg Tyr

1537 CTG GTC GCG CTT AAC TTC TCC GGC GAT GCG CAG GCA CTT GTT CTC CCG
Leu Val Ala Leu Asn Phe Ser Gly Asp Ala Gln Ala Leu Val Leu Pro

1585 ACA GAC CTG AGC GCC GCG TCA CCT GTT ATC GGG CGC GCC CCG CAA GTG
Thr Asp Leu Ser Ala Ala Ser Pro Val Ile Gly Arg Ala Pro Gln Val

1633 GAC CGC ATG CAG CAT GAT GCT GCA CGG ATC GAG CTG ATG GGT TGG GAA
Asp Arg Met Gln His Asp Ala Ala Arg Ile Glu Leu Met Gly Trp Glu

1681 GCG CGG GTC TAC CAC TGC GCA TGA
Ala Arg Val Tyr His Cys Ala ---

## Patentansprüche

1. DNA-Sequenz,
**dadurch gekennzeichnet,**
**dass** sie für ein Protein mit einer Saccharose-Isomerase-Aktivität codiert und
(a) eine der in SEQ ID NO. 1, SEQ ID NO. 2, SEQ ID NO. 3, SEQ ID NO. 9, SEQ ID NO. 11 oder SEQ ID NO. 13 gezeigten Nukleotidsequenzen gegebenenfalls ohne den Signalpeptid-codierenden Bereich,
(b) eine der Sequenzen aus (a) im Rahmen der Degeneration des genetischen Codes entsprechende Nukleotidsequenz, oder
(c) eine mit den Sequenzen aus (a) oder/und (b) hybridisierende Nukleotidsequenz umfasst, wobei die Hybridisierung nach Waschen für 1 h mit 1x SSC und 0,1 % SDS bei 55 °C erfolgt.

2. DNA-Sequenz nach Anspruch 1,
**dadurch gekennzeichnet,**
**daß** sie
(a) eine der in SEQ ID NO. 1, SEQ ID NO. 2, SEQ ID NO. 3, SEQ ID NO. 9, SEQ ID NO. 11 oder SEQ ID NO. 13 gezeigten Nukleotidsequenzen gegebenenfalls ohne den Signalpeptid-codierenden Bereich oder
(b) eine zu den Sequenzen aus (a) mindestens 70 % homo- loge Nukleotidsequenz umfaßt.

3. DNA-Sequenz nach Anspruch 1 oder 2,
**dadurch gekennzeichnet,**
**daß** sie eine mindestens 80 % homologe Nukleotidsequenz zu den Teilbereichen von
(a) Nukleotid 139 - 155 oder/und
(b) Nukleotid 625 - 644
der in SEQ ID NO. 1 gezeigten Nukleotidsequenz aufweist.

4. DNA-Sequenz nach einem der Ansprüche 1 bis 3,
**dadurch gekennzeichnet,**
**daß** sie eine mindestens 80 % homologe Nukleotidsequenz zu den Teilbereichen von
(c) Nukleotid 995 - 1013 oder/und
(d) Nukleotid 1078 - 1094
der in SEQ ID NO. 1 gezeigten Nukleotidsequenz aufweist.

5. Vektor,
**dadurch gekennzeichnet,**
**daß** er mindestens eine Kopie einer DNA-Sequenz nach einem der Ansprüche 1 bis 4 enthält.

6. Vektor nach Anspruch 5,
**dadurch gekennzeichnet,**
**daß** er ein prokaryontischer Vektor ist.

7. Vektor nach Anspruch 5 oder 6,
**dadurch gekennzeichnet,**
**daß** er ein zirkuläres Plasmid ist.

8. Vektor nach Anspruch 6 oder 7,
**dadurch gekennzeichnet,**
**daß** er in einer Wirtszelle mit einer Kopienzahl von weniger als 10 vorliegt.

9. Vektor nach Anspruch 7 oder 8,
**dadurch gekennzeichnet,**
**daß** er das Saccharose-Isomerasegen unter Kontrolle eines regulierbaren Promotors enthält.

10. Vektor nach einem der Ansprüche 5-9, der das Plasmid pHWS 88 (DSM 8824) ist.

11. Zelle,
**dadurch gekennzeichnet,**
**daß** sie mit einer DNA-Sequenz nach einem der Ansprüche 1 bis 4 oder einem Vektor nach einem der Ansprüche 5 bis 10 transformiert ist.

12. Zelle nach Anspruch 11,
**dadurch gekennzeichnet,**
**daß** sie eine prokaryontische Zelle ist.

13. Zelle nach Anspruch 12,
**dadurch gekennzeichnet,**
**daß** sie eine gram-negative prokaryontische Zelle ist.

14. Zelle nach Anspruch 12,
**dadurch gekennzeichnet,**
**daß** sie eine Enterobakterienzelle ist.

15. Zelle nach Anspruch 12,
**dadurch gekennzeichnet,**
**daß** sie eine Escherichia coli-, Protaminobacter rubrum- oder Erwinia rhapontici-Zelle ist.

16. Protein mit einer Saccharose-Isomerase-Aktivität,
**dadurch gekennzeichnet,**
**daß** es eine der in SEQ ID NO. 4, SEQ ID NO. 5 oder SEQ ID NO. 6, SEQ ID NO. 10, SEQ ID NO. 12 oder SEQ ID NO. 14 gezeigten Aminosäuresequenzen gegebenenfalls ohne den Signalpeptid- bereich umfaßt.

17. Zelle, die mindestens eine für ein Protein mit einer Saccharose-Isomerase-Aktivität codierende DNA-Sequenz nach einem der Ansprüche 1 bis 4 enthält und einen reduzierten Palatinose- oder/und Trehalulosestoffwechsel aufweist, und die bei Kultivierung in einem Saccharose enthaltenden Medium zu einer dauerhaften Akkumulierung von Palatinose in der Lage ist.

18. Zelle nach Anspruch 17,
**dadurch gekennzeichnet,**
**daß** die Reduzierung des Palatinose- oder/und Trehalulo- sestoffwechsels durch teilweise oder vollständige Hemmung der Expression von Invertaseoder/und Palatinasegenen erfolgt.

19. Protaminobacter rubrum Mutante SZZ 13 (DSM 9121)

20. Verfahren zur Isolierung von Nukleinsäuren, die für ein Protein mit einer Saccharose-Isomerase-Aktivität codieren,
**dadurch gekennzeichnet,**
**daß** man eine Genbank aus einem Spenderorganismus, der eine für ein Protein mit einer Saccharose-Isomerase-Aktivität codierende DNA-Sequenz enthält, in einem geeigneten Wirtsorganismus anlegt, die Klone der Genbank untersucht unter Verwendung von Nukleinsäuresonden, die aus den Sequenzen SEQ ID NO. 1, SEQ ID NO. 2, SEQ ID NO. 3, SEQ ID NO. 9, SEQ. ID NO.11 oder SEQ ID NO. 13 abgeleitet sind, und die Klone isoliert, die eine für ein Protein mit Saccharose-Isomerase-Aktivität codierende Nukleinsäure enthalten.

21. Verfahren nach Anspruch 20,
**dadurch gekennzeichnet,**
**daß** man als Wirtsorganismus E.coli verwendet.

22. Verfahren nach Anspruch 20 oder 21,
**dadurch gekennzeichnet,**
**daß** man als Nukleinsäuresonde ein DNA-Fragment verwendet, das durch PCR-Amplifikation der DNA aus dem Spenderorganismus unter Verwendung der Oligonukleotidgemische 5'-TGGTGGAA(A,G)GA(A,G)GCTGT-3' und 5'-TCCCAGTTCAG(A,G)TCCGGCTG-3' als Primer erhalten wurde.

23. Verfahren zur Herstellung von nicht-kariogenen Zuckern, insbesondere Trehalulose oder/und Palatinose,
**dadurch gekennzeichnet,**
**daß** man zur Produktion der Zucker ein Protein nach Anspruch 16, einen Organismus nach einem der Ansprüche 11 bis 15 oder 17 bis 19 oder einen Extrakt aus einem derartigen Organismus verwendet.

24. Verfahren nach Anspruch 23,
**dadurch gekennzeichnet,**
**daß** man den Organismus,den Extrakt oder das Protein in immobilisierter Form verwendet.

25. Verwendung von Proteinen nach Anspruch 16, oder Organismen nach einem der Ansprüche 11 bis 15 oder 17 bis 19 zur Herstellung von nicht-kariogenen Zuckern, insbesondere Trehalulose oder/und Palatinose.

## Claims

1. DNA sequence **characterized in that** it codes for a protein with a sucrose isomerase activity and comprises
(a) one of the nucleotide sequences shown in SEQ ID NO. 1, SEQ ID NO. 2, SEQ ID NO. 3, SEQ ID NO. 9, SEQ ID NO. 11 or SEQ ID NO. 13, where appropriate without the signal peptide-coding region,
(b) a nucleotide sequence corresponding to the sequences from (a) within the scope of the degeneracy of the genetic code, or
(c) a nucleotide sequence which hybridizes with the sequences from (a) and/or (b), the hybridization being performed after washing for 1h with 1 x ssc and 0.1% SDS at 5°C.

2. DNA sequence according to Claim 1, **characterized in that** it comprises
(a) one of the nucleotide sequences shown in SEQ ID NO. 1, SEQ ID NO. 2, SEQ ID NO. 3, SEQ ID NO. 9, SEQ ID NO. 11 or SEQ ID NO. 13, where appropriate without the signal peptide-coding region, or
(b) a nucleotide sequence which is at least 70% homologous with the sequences from (a).

3. DNA sequence according to Claim 1 or 2, **characterized in that** it has an at least 80% homologous nucleotide sequence to the part-regions of
(a) nucleotide 139 - 155 and/or
(b) nucleotide 625 - 644 of the nucleotide sequence shown in SEQ ID NO. 1.

4. DNA sequence according to any of Claims 1 to 3, **characterized in that** it has an at least 80% homologous nucleotide sequence to the part-regions of
(c)nucleotide 995 - 1013 and/or
(d)nucleotide 1078 - 1094
of the nucleotide sequence shown in SEQ ID NO. 1.

5. Vector **characterized in that** it contains at least one copy of a DNA sequence according to any of Claims 1 to 4.

6. Vector **characterized in that** it is a prokaryotic vector.

7. Vector according to Claim 5 or 6, **characterized in that** it is a circular plasmid.

8. Vector according to Claim 6 or 7, **characterized in that** it is present in a host cell with a copy number of less than 10.

9. Vector according to Claim 7 or 8, **characterized in that** it contains the sucrose isomerase gene under the control of a regulatable promoter.

10. Vector according to any of Claims 5-9 which is the plasmid pHWS 88 (DSM 8824).

11. Cell **characterized in that** it is transformed with a DNA sequence according to any of Claims 1 to 4 or with a vector according to any of Claims 5 to 10.

12. Cell according to Claim 11, **characterized in that** it is a prokaryotic cell.

13. Cell according to Claim 12, **characterized in that** it is a Gram-negative prokaryotic cell.

14. Cell according to Claim 12, **characterized in that** it is an enterobacterial cell.

15. Cell according to Claim 12, **characterized in that** it is an Escherichia coli, Protaminobacter rubrum or Erwinia rhapontici cell.

16. Protein with a sucrose isomerase activity, **characterized in that** it comprises one of the amino-acid sequences shown in SEQ ID NO. 4, SEQ ID NO. 5 or SEQ ID NO. 6, SEQ ID NO. 10, SEQ ID NO. 12 or SEQ ID NO. 14, where appropriate without the signal peptide region.

17. Cell which contains at least one DNA sequence according to any of Claims 1 to 4 coding for a protein with a sucrose isomerase activity and has a reduced palatinose and/or trehalulose metabolism, and which on cultivation in a sucrose-containing medium is capable of continuous accumulation of palatinose.

18. Cell according to Claim 17, **characterized in that** the reduction of the palatinose and/or trehalulose metabolism takes place by partial or complete inhibition of the expression of invertase and/or palatinase genes.

19. Protaminobacter rubrum mutant SZZ 13 (DSM 9121).

20. Method for isolating nucleic acids which code for a protein with a sucrose isomerase activity, **characterized in that** a gene bank from a donor organism which contains a DNA sequence coding for a protein with a sucrose isomerase activity is set up in a suitable host organism, the clones of the gene bank are examined using nucleic acid probes derived from the sequences SEQ ID NO. 1, SEQ ID NO. 2, SEQ ID NO. 3, SEQ ID NO. 9, SEQ ID NO. 11 or SEQ ID NO. 13, and the clones which contain a nucleic acid coding for a protein with sucrose isomerase activity are isolated.

21. Process according to Claim 20, **characterized in that** E. coli is used as host organism.

22. Process according to Claim 20 or 21,
**characterized in that** a DNA fragment which has been obtained by PCR amplification of the DNA from the donor organism using the oligonucleotide mixtures 5'-TGGTGGAA (A,G)GA(A,G)GCTGT-3' and 5'-TCCCAGTTCAG(A,G)TCCGGCTG-3' as primers is used as nucleic acid probe.

23. Process for the production of non-cariogenic sugars, in particular trehalulose and/or palatinose, **characterized in that** a protein according to Claim 16, an organism according to any of Claims 11 to 15 or 17 to 19 or an extract from an organism of this type is used for the production of the sugars.

24. Process according to Claim 23, **characterized in that** the organism, the extract or the protein is used in immobilized form.

25. Use of proteins according to Claim 16 or organisms according to any of Claims 11 to 15 or 17 to 19 for the production of non-cariogenic sugars, in particular trehalulose and/or palatinose.

## Revendications

1. Séquence d'ADN
**caractérisée en ce que**
elle code pour une protéine ayant une activité saccharose isomérase et comporte
(a) une des séquences de nucléotides reproduites dans les séquences SEQ ID NO. 1, SEQ ID NO. 2, SEQ ID NO. 3, SEQ ID NO. 9, SEQ ID NO. 11 ou SEQ ID NO. 13, le cas échéant sans le domaine codant pour le peptide signal,
(b) une des séquences de nucléotides correspondant aux séquences de (a) dans le cadre de la dégénérescence du code génétique ou
(c) une séquence de nucléotides qui s'hybride avec les séquences de (a) ou/et de (b), l'hybridation intervenant après un lavage pendant 1 h avec 1 x SSC et 0,1 % de SDS à 55°C.

2. Séquence d'ADN selon la revendication 1,
**caractérisée en ce que**
elle comporte
(a) une des séquences de nucléotides reproduites dans les séquences SEQ ID NO. 1, SEQ ID NO. 2, SEQ ID NO. 3, SEQ ID NO. 9, SEQ ID NO. 11 ou SEQ ID NO. 13, le cas échéant sans le domaine codant pour le peptide signal ou
(b) une séquence de nucléotides homologue à au moins 70% aux séquences de (a).

3. Séquence d'ADN selon la revendication 1 ou 2,
**caractérisée en ce que**
elle présente une séquence de nucléotides homologue à au moins 80 % aux domaines partiels
(a) des nucléotides 139 - 155 et/ou
(b) des nucléotides 625 - 644 de la séquence de nucléotides reproduite dans SEQ ID NO. 1.

4. Séquence d'ADN l'une quelconque des revendications 1 à 3,
**caractérisée en ce que**
elle présente une séquence de nucléotides homologue à au moins 80 % aux domaines partiels
(a) des nucléotides 995 - 1013 et/ou
(b) des nucléotides 1078 - 1094 de la séquence de nucléotides reproduite dans SEQ ID NO. 1.

5. Vecteur,
**caractérisé en ce que**
il contient au moins une copie d'une séquence d'ADN selon l'une quelconque des revendications 1 à 4.

6. Vecteur selon la revendication 5,
**caractérisé en ce que**
c'est un vecteur procaryote.

7. Vecteur selon la revendication 5 ou 6
**caractérisé en ce que**
c'est un plasmide circulaire.

8. Vecteur selon la revendication 6 ou 7,
**caractérisé en ce que**
il est présent dans une cellule hôte à un nombre de copies inférieur à 10.

9. Vecteur selon la revendication 7 ou 8,
**caractérisé en ce que**
il contient le gène de la saccharose isomérase sous le contrôle d'un promoteur régulable.

10. Vecteur selon l'une quelconque des revendications 5-9 qui est le plasmide pHWS 88 (DSM 8824).

11. Cellule
**caractérisée en ce que**
elle est transformée avec une séquence d'ADN selon l'une quelconque des revendications 1 à 4 ou un vecteur selon l'une quelconque des revendications 5 à 10.

12. Cellule selon la revendication 11,
**caractérisée en ce que**
c'est une cellule procaryote.

13. Cellule selon la revendication 12,
**caractérisée en ce que**
c'est une cellule procaryotique Gram-négative.

14. Cellule selon la revendication 12,
**caractérisée en ce que**
c'est une cellule entérobactérienne.

15. Cellule selon la revendication 12,
**caractérisée en ce que**
c'est une cellule d'Escherichia coli, de Protaminobacter rubrum ou d'Erwinia rhapontici.

16. Protéine ayant une activité saccharose isomérase,
**caractérisée en ce que**
elle contient une des séquences d'acides aminés reproduites dans les séquences SEQ ID NO. 4, SEQ ID NO. 5, SEQ ID NO. 6, SEQ ID NO. 10, SEQ ID NO. 12 ou SEQ ID NO. 14, le cas échéant sans le domaine de peptide signal.

17. Cellule qui contient au moins une séquence d'ADN codant pour une protéine ayant une activité saccharose isomérase selon l'une quelconque des revendications 1 à 4 et présente un métabolisme réduit du palatinose et/ou du tréhalulose et qui est en mesure d'accumuler durablement du palatinose lors de la culture dans un milieu contenant du saccharose.

18. Cellule selon la revendication 17,
**caractérisée en ce que**
la réduction du métabolisme du palatinose et/ou du tréhalulose intervient par inhibition partielle ou complète de l'expression de gènes d'invertase et/ou de palatinase.

19. Mutants SZZ 13 (DSM 9121 ) de Protaminobacter rubrum

20. Procédé d'isolement d'acides nucléiques codant pour une protéine ayant une activité saccharose isomérase,
**caractérisé en ce que**
on crée une banque de gènes à partir d'un organisme donneur qui contient une séquence d'ADN codant pour une protéine ayant une activité saccharose isomérase, dans un organisme hôte approprié, on étudie les clones de la banque de gènes en utilisant des sondes d'acides nucléiques qui sont dérivées des séquences SEQ ID NO. 1, SEQ ID NO. 2, SEQ ID NO. 3, SEQ ID NO. 9, SEQ ID NO. 11 ou SEQ ID NO. 13 et on isole les clones qui contiennent un acide nucléique codant pour une protéine ayant une activité saccharose isomérase.

21. Procédé selon la revendication 20,
**caractérisé en ce que**
on utilise E. coli comme organisme hôte.

22. Procédé selon la revendication 20 ou 21,
**caractérisé en ce que**
on utilise, comme sonde d'acide nucléique, un fragment d'ADN que l'on a obtenu par amplification PCR de l'ADN de l'organisme donneur en utilisant les mélanges d'oligonucléotides 5'-TGGTGGAA(A,G)GA(A,G) GCTGT-3' et 5'-TCCCAGTTCAG(A,G)TCCGGCTG-3' en tant qu'amorce.

23. Procédé de fabrication de sucres acariogènes, en particulier de tréhalulose et/ou de palatinose,
**caractérisé en ce que**
on utilise, pour produire les sucres, une protéine selon la revendication 16, un organisme selon l'une quelconque des revendications 11 à 15 ou 17 à 19 ou un extrait d'un tel organisme.

24. Procédé selon la revendication 23,
**caractérisé en ce que**
on utilise l'organisme, l'extrait ou la protéine sous forme immobilisée.

25. Utilisation de protéines selon la revendication 16 ou d'organismes selon l'une quelconque des revendications 11 à 15 ou 17 à 19 pour fabriquer des sucres acariogènes, en particulier du tréhalulose et/ou du palatinose.
